# EUROPÄISCHE PATENTSCHRIFT

(11) **EP 0 503 410 B1**
(45) Veröffentlichungstag und Bekanntmachung des Hinweises auf die Patenterteilung: **26.06.1996**
(21) Anmeldenummer: 92103513.5
(22) Anmeldetag: 29.02.1992
(51) Int. Cl.: C07D 261/18, C07D 275/02, A01N 43/80, C07D 413/04, C07D 413/12, C07F 7/08, C07F 9/653, C07F 9/6539

(54) **Isoxazol- und Isothiazol-5-carbonsäureamide**
Isoxazol- and Isothiazol 5-carboxylic acids amids
Amides d'acides isoxazole et isothiazole-5-carboxyliques

(30) Priorität: 14.03.1991 DE 4108181
(43) Veröffentlichungstag der Anmeldung: 16.09.1992
(73) Patentinhaber: BASF Aktiengesellschaft, D-67063 Ludwigshafen (DE)
(72) Erfinder: Maywald, Volker, Dr., W-6700 Ludwighafen (DE); Muenster, Peter, Dr., W-6823 Neulussheim (DE); Koenig, Hartmann, Dr., W-6703 Limburgerhof (DE); Hamprecht, Gerhard, Dr., W-6940 Weinheim (DE); Kuekenhoehner, Thomas, Dr., W-6737 Boehl-Iggelheim (DE); Walter, Helmut, Dr., W-6719 Obrigheim (DE); Westphalen, Karl-Otto, Dr., W-6720 Speyer (DE); Gerber, Matthias, Dr., W-6704 Mutterstadt (DE)

(56) Entgegenhaltungen:
- EP-A- 0 193 131
- EP-A- 0 337 263
- EP-A- 0 418 667

## Beschreibung

Die vorliegende Erfindung betrifft Carbonsäureamide der Formel I 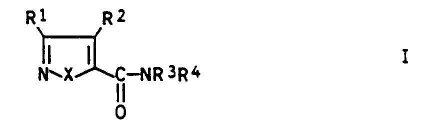
in der die Variablen folgende Bedeutung haben:
X
Sauerstoff oder Schwefel;
R¹
Halogen,
C₁-C₄-Alkylthio, C₁-C₄-Halogenalkoxy, C₁-C₄-Halogenalkylthio, Cyano, Formyl, C₂-C₄-Alkanoyl, C₁-C₄-Alkoxycarbonyl, C₁-C₄-Alkylaminocarbonyl, Di-(C₁-C₃)-alkylaminocarbonyl, Cyclopropylaminocarbonyl, Methylsulfonyl, Trifluormethylsulfonyl;
Phenoxy oder Phenylthio, welches bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein kann;
R²
eine Formylgruppe, eine Carbonsäurehalogenidgruppe, eine 4,5-Dihydrooxazol-2-ylgruppe, ein Rest COYR⁵ wobei die Variablen die folgende Bedeutung haben:
Y
Sauerstoff oder Schwefel;
R⁵
Wasserstoff;
eine C₁-C₆-Alkylgruppe, welche ein bis fünf Halogenatome und/oder bis zu drei Hydroxy- und/oder C₁-C₄-Alkoxygruppen und/oder einen der folgenden Reste tragen kann:
- Cyano,
- C₁-C₄-Alkoxy-C₂-C₄-alkoxy,
- C₁-C₃-Alkylthio,
- C₁-C₃-Alkylamino, Di-(C₁-C₃)-alkylamino, C₃-C₆-Cycloalkylamino oder Di-(C₃-C₆)-cycloalkylamino,
- Trimethylsilyl,
- C₁-C₃-Alkylsulfinyl oder C₁-C₃-Alkylsulfonyl,
- Carboxyl, C₁-C₃-Alkoxycarbonyl, C₁-C₃-Alkoxycarbonyl-C₁-C₃-alkoxy oder C₁-C₃-Alkoxycarbonyl-C₁-C₃-alkoxy-C₁-C₃-alkoxycarbonyl,
- Di-(C₁-C₃)-alkylaminocarbonyl,
- Di-(C₁-C₃)-alkoxyphosphonyl,
- C₁-C₆-Alkaniminoxy oder C₅-C₆-Cycloalkaniminoxy,
- N-Phthalimido, N-Succinimido, Benzyloxy, Benzoyl, wobei diese cyclischen Reste zusätzlich eine bis drei der folgenden Gruppen tragen können: Halogen, C₁-C₃-Alkyl oder C₁-C₃-Alkoxy,
- einen 5- oder 6-gliedrigen gesättigten heterocyclischen Rest oder einen 5- oder 6-gliedrigen heteroaromatischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sind und wobei die Heterocyclen noch einen oder zwei der folgenden Substituenten tragen können: Halogen, C₁-C₃-Alkyl, C₁-C₃-Alkoxy oder C₁-C₃-Alkoxycarbonyl,
- Phenyl, das noch bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy oder C₁-C₂-alkoxycarbonyl;
- einen Rest -CR¹⁰=N-R¹¹, wobei R¹⁰ und R¹¹ die folgende Bedeutung haben:
R¹⁰
Wasserstoff oder C₁-C₆-Alkyl und
R¹¹
C₁-C₆-Alkoxy, C₃-C₆-Alkenyloxy oder C₃-C₆-Alkinyloxy, die jeweils 1 bis 3 Halogenatome und/oder einen Phenylrest mit gewünschtenfalls eins bis drei der folgenden Reste tragen können: Halogen, Nitro, Cyano, C₁-C₃-Alkyl, C₁-C₃-Alkoxy; Phenoxy, das noch bis zu drei der folgenden Substituenten tragen kann: Halogen, Nitro, Cyano, C₁-C₃-Alkyl oder C₁-C₃-Alkoxy; C₁-C₆-Alkylamino, Di-(C₁-C₆)-alkylamino oder Phenylamino, wobei der Phenylrest zusätzlich ein bis drei der folgenden Reste tragen kann: Halogen, Nitro, Cyano, C₁-C₃-Alkyl oder C₁-C₃-Alkoxy;
R⁵ ferner:
C₃-C₈-Cycloalkyl;
C₃-C₆-Alkenyl, C₅-C₆-Cycloalkenyl, C₃-C₆-Alkinyl, wobei diese Reste eine der folgenden Gruppen tragen können: Hydroxy, Halogen, C₁-C₄-Alkoxy oder Phenyl, wobei der Aromat seinerseits eine bis drei der folgenden Gruppen tragen kann: Halogen, Nitro, Cyano, C₁-C₄-Alkyl, partiell oder vollständig halogeniertes C₁-C₄-Alkyl oder C₁-C₄-Alkoxy;
Phenyl, das eine bis drei der folgenden Gruppen tragen kann: Halogen, Nitro, Cyano, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkoxycarbonyl;
einen fünf- oder sechsgliedrigen heterocyclischen Rest mit bis zu drei Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, C₁-C₃-Alkyl, C₁-C₃-Alkoxy oder C₁-C₃-Alkoxycarbonyl;
einen Benzotriazolrest;
N-Phthalimido, Tetrahydrophthalimido, Succinimido, Maleinimido;
die 2,2-Dimethyl-1,3-dioxolan-4-ylmethyl- oder 1,3-Dioxolan-2-on-4-yl-methylgruppe;
im Falle Y = O: ein Äquivalent eines Kations aus der Gruppe der Alkali- und Erdalkalimetalle, Mangan, Kupfer, Eisen, Ammonium und mit bis zu 4 C₁-C₃-Alkylgruppen substituiertes Ammonium; oder
ein Rest -N=CR⁸R⁹, wobei
R⁸, R⁹
Wasserstoff; C₁-C₄-Alkyl, das unsubstituiert oder partiell oder vollständig halogeniert sein und einen C₁-C₃-Alkoxy- oder Phenylrest tragen kann, wobei der Phenylrest seinerseits noch ein bis dreimal durch Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy substituiert sein kann; C₃-C₆-Cycloalkyl; C₁-C₄-Alkoxy; Furanyl oder Phenyl, das zusätzlich bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy; oder R⁸ und R⁹ gemeinsam eine Methylenkette mit 4 bis 7 Gliedern bedeuten;
einen Rest -W-Z, wobei W eine C₂-C₄-Alkylenkette, eine Ethoxyethylenkette, eine But-2-enylen- oder eine But-2-inylenkette bedeutet und Z einen in ω-Stellung an W gebundenen Molekülteil, der den gleichen Molekülteil darstellt, der in α-Stellung von W mit W verknüpft ist, bedeutet;
R³
Wasserstoff;
C₁-C₆-Alkyl, das einen bis drei der folgenden Substituenten tragen kann: Hydroxy, Halogen, C₁-C₄-Alkoxy, C₁-C₄-Alkylthio oder Di-(C₁-C₄)-alkylamino;
C₃-C₈-Cycloalkyl, das ein- bis dreimal durch Halogen, C₁-C₄-Alkyl und partiell oder vollständig halogeniertes C₁-C₄-Alkyl substituiert sein kann;
R⁴
Wasserstoff; Hydroxyl, eine C₁-C₄-Alkoxygruppe;
eine C₁-C₆-Alkylgruppe, die einen bis drei der folgenden Reste tragen kann: Halogen, Cyano, C₁-C₄-Alkoxy, partiell oder vollständig halogeniertes C₁-C₄-Alkoxy, C₁-C₄-Alkylthio, partiell oder vollständig halogeniertes C₁-C₄-Alkylthio, Di-C₁-C₄-alkylamino, C₃-C₈-Cycloalkyl oder Phenyl, wobei der Phenylring seinerseits einen bis drei der folgenden Reste tragen kann: Halogen, Cyano, Nitro, C₁-C₄-Alkyl, partiell oder vollständig halogeniertes C₁-C₄-Alkyl, C₁-C₄-Alkoxy, C₁-C₄-Alkylthio oder partiell oder vollständig halogeniertes C₁-C₄-Alkoxy oder C₁-C₄-Alkylthio;
eine C₃-C₈-Cycloalkylgruppe, die einen bis drei der folgenden Reste tragen kann: Halogen, Nitro, Cyano, C₁-C₆-Alkyl, partiell oder vollständig halogeniertes C₁-C₆-Alkyl, C₁-C₄-Alkoxy oder partiell oder vollständig halogeniertes C₁-C₄-Alkoxy;
eine C₃-C₆-Alkenylgruppe, deren Doppelbindung epoxidiert sein kann, oder eine C₃-C₆-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
eine Di-(C₁-C₄)-alkylaminogruppe;
ein 5- bis 6-gliedriger heterocyclischer gesättigter oder aromatischer Rest mit einem oder zwei Heteroatomen, ausgewählt aus der Gruppe Sauerstoff, Schwefel und Stickstoff, der ein- bis dreimal durch C₁-C₄-Alkyl oder Halogen substituiert sein kann;
eine Phenylgruppe, die eine bis vier der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Nitro, Cyano, Formyl, C₂-C₄-Alkanoyl, C₂-C₄-Halogenalkanoyl oder C₁-C₄-Alkoxycarbonyl;
eine Naphthylgruppe, die ein- bis dreimal durch C₁-C₄-Alkyl oder Halogen substituiert sein kann;
oder
R³, R⁴
gemeinsam eine Methylenkette mit 4 bis 7 Gliedern, welche durch Sauerstoff, Schwefel oder N-Methyl unterbrochen sein kann, oder den Rest -(CH₂)₃-CO-
sowie die umweltverträglichen Salze der Verbindungen I.

Außerdem betrifft die Erfindung herbizide Mittel, welche die Verbindungen I als wirksame Substanzen enthalten und Verfahren zur Herstellung der Verbindungen I.

Herbizid wirksame Isoxazol- und Isothiazol-5-carbonsäureamide bzw. deren Derivate sind bekannt, z.B. aus DE-A 38 12 225. Trotz der an sich guten herbiziden Wirksamkeit dieser Verbindungen bestand die Aufgabe ähnliche Verbindungen mit verbesserter Kulturpflanzenselektivität bereitzustellen.

Demgemäß wurden die eingangs definierten Isoxazol- und Isothiazol-5-carbonsäureamide gefunden.

Die erfindungsgemäßen Carbonsäureamide der Formel I sind auf verschiedenen Wegen herstellbar und zwar vorzugsweise nach den folgenden Verfahren:
1. Ein Verfahren zur Synthese der erfindungsgemäßen Isoxazol- bzw. Isothiazol-5-carboxamide der Formel I, in der R² Carboxyl bedeutet, geht von in 3-Position verschieden substituierten Isoxazol- bzw. Isothiazol-4,5-dicarbonsäuredialkylestern der Formel II aus. Diese werden zunächst mit einem Äquivalent einer wäßrigen Base zu den Monocarbonsäuren III hydrolysiert, die in bekannter Weise in die Halogenide IV oder andere aktivierte Formen der Carbonsäure überführt und anschließend mit einem Amin Va amidiert werden. Anschließend wird die 4-Carbonsäureestergruppe in bekannter Weise verseift. Dieses Verfahren wurde bereits in der DE-A 38 12 225 ausführlich beschrieben. 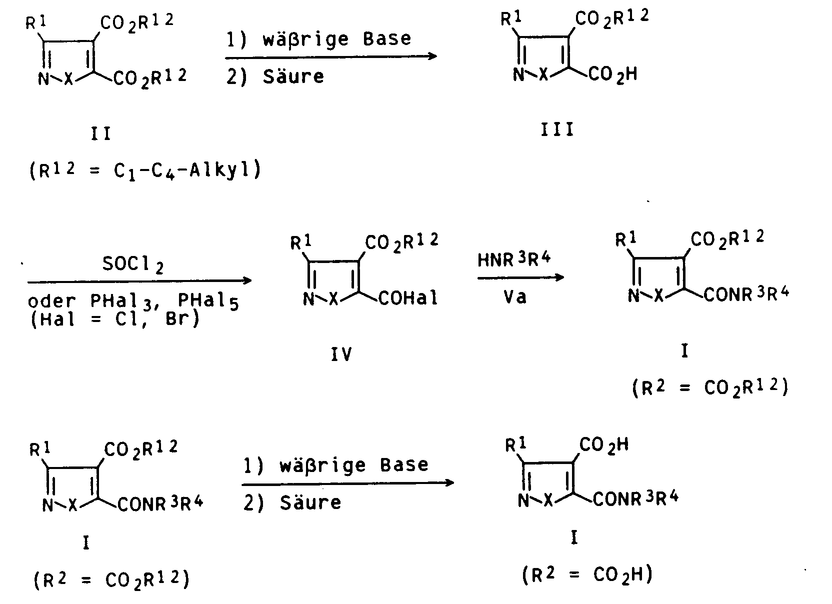 Durch Veresterung der Carbonsäure (I mit R² = CO₂H) oder Halogenierung in an sich bekannter Weise gelangt man zu den entsprechenden Säurederivaten.
   Die für dieses Verfahren benötigten Isoxazol- bzw. Isothiazol-4,5-dicarbonsäureesterdialkylester II sind entweder literaturbekannt, auf literaturbekannten Wegen herstellbar oder beispielsweise auf folgenden Wegen zugänglich:
   a) Ein Verfahren zur Herstellung von Isoxazol-4,5-dicarbonsäuredialkylestern der Formel II, in der R¹=Halogen bedeutet, besteht darin, daß man ein Dihalogenformaldoxim VI vorzugsweise Dibromo- bzw. Dichloroformaldoxim in Anwesenheit einer Base mit Acetylendicarbonsäuredialkylestern VII zu den entsprechenden 3-Halogen-isoxazol-4,5-dicarbonsäurediestern II umsetzt. Dazu geht man vorzugsweise so vor, daß man das Dihalogenformaldoxim VI in einem Lösungsmittel zu einer Mischung aus einem bis zehn Äquivalenten Acetylendicarbonsäuredialkylester VII und einem ebenfalls ein- bis zehnfachen molarem Überschuß einer Base, ebenfalls gelöst in einem Lösungsmittel, einem Lösungsmittelgemisch oder einem Zweiphasensystem aus einem organischen Lösungsmittel und Wasser, zutropft und nach einer Reaktionszeit von zwei bis zwanzig Stunden aufarbeitet. 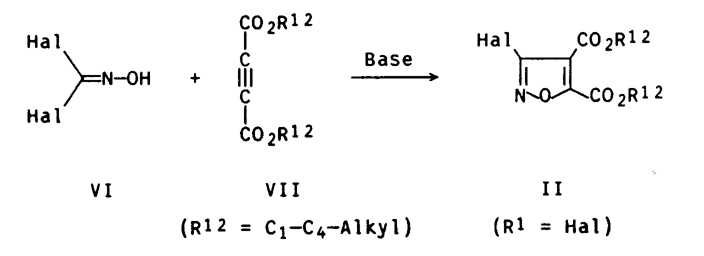
      Geeignete Lösungsmittel für diese Reaktion sind z.B. Alkohole wie Methanol, Ethanol, Propanol oder Isopropanol, Ether wie Diethylether, Methyl-tert.-butylether, Tetrahydrofuran oder Dioxan, Kohlenwasserstoffe wie Pentan, Hexan, Cyclohexan, Petrolether oder Ligroin, halogenierte aliphatische Kohlenwasserstoffe wie Methylenchlorid, Chloroform, Tetrachlormethan, Dichlorethan, Trichlorethan, Tetra- oder Hexachlorethan, aromatische Verbindungen wie Benzol, Toluol, Xylole oder Chlorbenzole, Ester wie Ethylacetat sowie Formamid, N-Methylpyrrolidon, Dimethylsulfoxid und Sulfolan.
      Die Temperatur, bei der die Reaktion durchgeführt wird, kann im Bereich von -15°C bis zum Siedepunkt des Lösungsmittels variiert werden, besonders bevorzugt ist der Bereich von 0 bis 40°C. Die Reaktion kann auch bei erhöhtem Druck ausgeführt werden, bevorzugt ist aber Atmosphärendruck.
      Als Basen dienen organische Verbindungen wie aliphatische oder aromatische Amine oder Pyridine, bevorzugt sind aber anorganische Verbindungen wie die Carbonate und Hydrogencarbonate der Alkali- und Erdalkalimetalle.
      Die für dieses Verfahren benötigten Aldoxime sind bekannt (D.M. Vyas, Y. Chiang, T.W. Doyle, Tetrahedron Lett., 25(5), p. 387 (1984), K. Hallig, I. Thomsen, K.B.G. Torssell, Liebigs Ann. Chem. 1989, 985 - 990).
   b) Isoxazol-4,5-dicarbonsäuredialkylester der Formel II, in der R¹ Cyano, C₁-C₄-Alkylthio, gegebenenfalls substituiertes Phenoxy oder gegebenenfalls substituiertes Thiophenyl bedeuten, erhält man beispielsweise dadurch, daß man ein Halogenisoxazol-4,5-dicarbonsäuredialkylester der Formel II, in der R¹ Chlor oder Brom bedeutet, mit Cyanidionen oder mit Alkoholaten bzw. Thiolaten der Formel IX umsetzt. Dazu geht man zweckmäßigerweise so vor, daß man mit einer geeigneten Base in einem inerten aprotisch polaren Lösungsmittel aus den entsprechenden Alkoholen oder Thiolen VIII

      R¹³XH VIII,

      in denen X für 0 oder S steht und R¹³ C₁-C₄-Alkylthio, ggf. substituiertes Phenoxy, ggf. substituiertes Thiophenyl bedeutet, Anionen vom Typ IX erzeugt und diese dann mit dem 3-Halogenisoxazol-4,5-dicarbonsäuredialkylester II in einem inerten dipolar aprotischen Lösungsmittel zur Reaktion bringt. Die Reihenfolge der Zugabe ist beliebig, in der Regel wird der Diester vorgelegt und das Anion in Lösung zugetropft. Die Umsetzung erfolgt im allgemeinen bei Temperaturen zwischen -20°C und der Siedetemperatur des Lösungsmittels, vorzugsweise bei 0 bis 50°C. 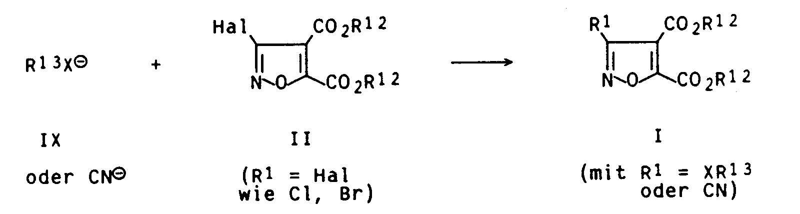 Geeignete Lösungsmittel sind beispielsweise Ether wie Diethylether, tert.-Butylmethylether, Tetrahydrofuran, Dioxan und 1,2-Dimethoxyethan.
      Als Base zur Freisetzung des Anions IX eignen sich Alkalimetallhydride wie Natrium- und Kaliumhydrid, Alkalimetallkohlenwasserstoffe wie n-Butyllithium, tert.-Butyllithium, Methyllithium und Phenyllithium oder bevorzugt bei acideren Alkoholen oder Thiolen Aminbasen wie Triethylamin, Pyridin, Ethyldicyclohexylamin, 1,4-Diazabicyclo[2.2.2]octan (DABCO) und 1,8-Diazabicyclo[5.4.0]undec-7-en (DBU).
      Die Komponenten (Alkohol, Thiol, Cyanid); Base; 3-Halogenisoxazol-4,5-dicarbonsäuredialkylester werden bevorzugt im Verhältnis 1:1:1 eingesetzt.
   c) Isoxazol-4,5-dicarbonsäuredialkylester der Formel II, in der R¹ Methylsulfonyl und Trifluormethylsulfonyl bedeutet, erhält man beispielsweise dadurch, daß man einen Isoxazol-4,5-dicarbonsäuredialkylester der Formel II, in der R¹ Methylthio oder Trifluormethylthio bedeutet, oxidiert. Die Überführung von Thioethern in Sulfone mit Oxidationsmitteln wie z.B. H₂O₂, Persäuren, Ozon, Kaliumpermanganat, Rutheniumtetroxid etc. ist eine allgemein bekannte Reaktion (A.C. Cope, D.E. Morrison, L. Field, J. Am. Chem. Soc. 72, p. 59 (1950); C.G. Overberger et. al., J. Am. Chem. Soc. 72, p. 2856 (1950); B.R. Baker, M.V. Querry, A.F. Kadish, J. Org. Chem. 15, 402 (1950); S.J. Daum, R.L. Clarke, THL 1967, 165; C. Djerassi, R.R. Engle, J. Am. Chem. Soc. 75, 3838 (1953); J. Goerdeler, M. Budnowski, Ber. Deutsch Chem. Ges. 94, p. 1682 (1961), Jerry March, Advanced Organic Chemistry, Third Edition, John Wiley & Sons 1985, p. 1089).
   d) Ein sehr breit anwendbares Verfahren zur Synthese verschieden substituierter Isoxazol-4,5-dicarbonsäuredimethylester der Formel II, in der R¹ beispielsweise Wasserstoff, gegebenenfalls substituiertes Alkyl, Cycloalkyl, Alkenyl, Cycloalkenyl, Alkinyl, Phenyl oder Heterocyclyl bedeutet, besteht darin, daß man ein entsprechend substituiertes Aldoxim der Formel X in Anwesenheit von Hypohalogenit mit Acetylendicarbonsäuredialkylester VII umsetzt. Bei dieser Reaktion wird das Aldoxim X durch das Hypohalogenit zum entsprechenden Nitriloxid oxidiert. Das Nitriloxid ist ein reaktiver 1,3-Dipol, der mit dem im Reaktionsmedium vorliegenden Dipolarophil Acetylendicarbonsäuredialkylester VII in einer Cycloaddition abgefangen wird.
      Zweckmäßigerweise werden äquimolare Mengen des Aldoxims X und des Acetylendicarbonsäure-diesters VII mit dem Hypohalogenit umgesetzt. Das Hypohalogenit kann in stöchiometrischer Menge zur Reaktionsmischung gegeben werden, in der Regel wird es jedoch in leicht überschüssiger Menge, bis zu einem zweifachen Überschuß, zum Reaktionsansatz dosiert. Aus verfahrenstechnischen Gründen kann es ggf. vorteilhaft sein, den Umsatz durch Verwendung unterstöchiometrischer Mengen an Hypohalogenit - etwa 50 bis 90 mol-% Hypohalogenit pro mol X - zu begrenzen. Ebenso ist es möglich, mit unter- oder überstöchiometrischen Mengen der Reaktanten X oder VII zu arbeiten.
      Als Hypohalogenite werden im allgemeinen Hypobromite und Hypochlorite, letztere bevorzugt, verwendet. Es können zu diesem Zweck wäßrige Lösungen der unterchlorigen oder unterbromigen Säure eingesetzt werden, vorzugsweise werden aber Alkalimetall- oder Erdalkalimetallhypochlorite oder - hypobromite, beispielsweise Natriumhypochlorit, Kaliumhypochlorit, Calciumhypochlorit, Magnesiumhypochlorit, Strontiumhypochlorit, Bariumhypochlorit oder die entsprechenden Hypobromite benutzt. Besonders bevorzugt werden Natrium-, Kalium- und Calciumhypochlorit und zwar in Form ihrer handelsüblichen, wäßrigen Lösungen angewandt.
      Geeignete Lösungsmittel für das Verfahren sind z.B. Alkohole, wie Methanol, Ethanol, Propanol oder Isopropanol, Ketone wie Aceton oder Methylethylketon, Ether wie Diethylether, Methyl-tert.-butylether, Tetrahydrofuran oder Dioxan, Kohlenwasserstoffe wie Pentan, Hexan, Cyclohexan, Petrolether, Weißöle oder Ligroin, halogenierte aliphatische Kohlenwasserstoffe wie Methylenchlorid, Chloroform, Tetrachlorkohlenstoff, Dichlorethan, Trichlorethan, Tetrachlorethan oder Perchlorethan, aromatische Verbindungen wie Benzol, Toluol, Xylole oder Chlorbenzole, Ester, wie Ethylacetat sowie Dimethylformamid, N-Methylpyrrolidon, Dimethylsulfoxid, Sulfolan usw.
      Die Temperatur, bei der die Umsetzung durchgeführt wird, kann in weiten Bereichen variiert werden. In der Regel findet die Umsetzung schon bei Temperaturen von (-15°C) und tiefer statt, und nach oben wird der Temperaturbereich im Prinzip nur durch den Siedepunkt des verwendeten Lösungsmittel begrenzt, da die Umsetzung zweckmäßigerweise bei Atmosphärendruck ausgeführt wird. Vorzugsweise wird bei Temperaturen im Bereich von 0 bis 40°C gearbeitet. Die Reaktion kann auch unter erhöhtem Druck ausgeführt werden, insbesondere unter autogen erzeugtem Druck.
      Die für dieses Verfahren benötigten Aldoxime X sind entweder bekannt oder können nach an sich bekannten Verfahren (z.B. Houben-Weyl, Methoden der organischen Chemie, Bd. 10/4, Seite 55 bis 56, Thieme Verlag, Stuttgart 1968) durch Umsetzung der entsprechenden Aldehyde mit Hydroxylamin, hergestellt werden. Die Aldoxime X können selbstverständlich sowohl in Form ihrer E- oder Z-Isomeren als auch als Gemische dieser Stereoisomeren verwendet werden. Die Acetylendicarbonsäure-diester VII sind im Handel erhältlich oder nach an sich bekannten Methoden (z.B. Organic Syntheses Coll. Vol 4, Seite 329) darstellbar. 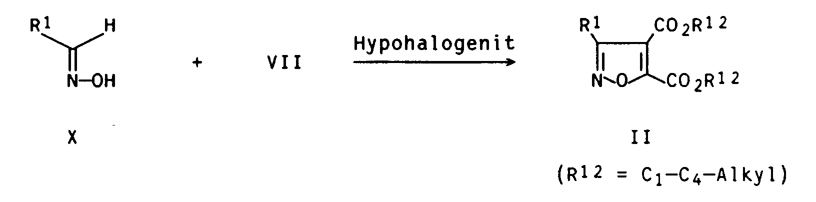
   e) Isoxazol-4,5-dicarbonsäuredimethylester der Formel II, in der R¹ Hydroxymethyl und R¹² Methyl bedeuten, erhält man dadurch, daß man zunächst unter den unter d) beschriebenen Bedingungen ausgehend von einem Aldoxim der Formel X, in der R¹ Acetoxymethyl bedeutet, in Anwesenheit von Natriumhypochlorit mit Acetylendicarbonsäuredimethylester VII (R¹²=Methyl) die Cycloaddition durchführt und anschließend die Acetoxygruppe hydrolytisch entfernt. Dazu geht man zweckmäßigerweise so vor, daß man den Acetoxymethyl-isoxazol-4,5-dicarbonsäuredimethylester II (R¹=Acetoxymethyl) in Methanol vorlegt und anschließend eine 1 bis 2fach molare Menge Natriummethylat zugibt. Die Reaktionstemperatur liegt bei 20 bis 65°C.
   f) Aus den Hydroxymethyl-substituierten-Isoxazol-4,5-dicarbonsäuredimethylestern sind die Formyl- oder Carboxyl-substituierten Verbindungen zugänglich, indem man die Hydroxymethylgruppe zur Aldehyd- oder Carboxygruppe oxidiert (Lit. Jerry March, Advanced Organic Chemistry, Third Edition, John Wiley & Sons 1985, p. 1048 bis 1120). Die Carboxylgruppe kann ferner auf übliche Art und Weise in die C₁-C₃-Alkoxycarbonyl-, die C₁-C₃-Aminocarbonyl-, und die Di-(C₁-C₃)-alkylaminocarbonylgruppe überführt werden.
2. Ein anderes Verfahren zur Herstellung von Verbindungen der Formel I, in der R¹ C₁-C₄-Alkylthio, Cyano, gegebenenfalls substituiertes Phenoxy oder gegebenenfalls substituiertes Thiophenyl und R² Carboxyl oder Formyl bedeuten, besteht darin, daß man die 3-Chlor-isoxazol-5-carbonsäure XI nach literaturbekannten Methoden amidiert und die so erhaltenen Amide XII nach Austausch von Chlorid durch eine entsprechende nucleophile Gruppe in an sich bekannter Weise baseninduziert in Gegenwart eines Carboxlierungs- oder Formylierungsreagenz umsetzt (vgl. DE-A 38 12 225). Der nukleophile Austausch des Chlorids wird unter den oben unter 1 b) beschriebenen Bedingungen durchgeführt. 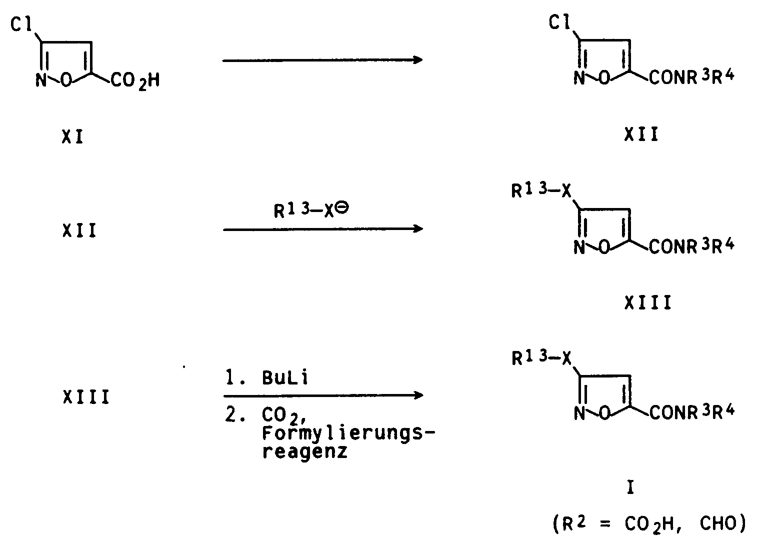
3. Verbindungen der Formel I in der R² COHal bedeutet, erhält man beispielsweise dadurch, daß man eine Carbonsäure der Formel I, in der R² COOH bedeutet, in üblicher Art und Weise mit einem anorganischen Säurechlorid wie Thionylchlorid, Phosphortri- oder Phosphorpentahalogeniden zur Reaktion bringt. Dabei wird zweckmäßigerweise das anorganische Säurehalogenid in 1 bis 5 Moläquivalenten, vorzugsweise 1 bis 2 Moläquivalenten, eingesetzt. Man kann ohne Lösungsmittel oder in Gegenwart eines inerten organischen Lösungsmittel wie z.B. Benzol oder Toluol bei Temperaturen zwischen Raumtemperatur und der Siedetemperatur des anorganischen Säurehalogenids bzw. des inerten organischen Lösungsmittels arbeiten. In manchen Fällen kann der Zusatz eines Katalysators wie Dimethylformamid oder 4-Dimethylaminopyridin von Vorteil sein. Nach Beendigung der Reaktion kann das Säurehalogenid auf übliche Art und Weise isoliert werden, z.B. durch Abdestillation des Überschusses an anorganischem Säurechlorid und des organischen Lösungsmittels.
4. Verbindungen der Formel I in der R² für eine Formylgruppe steht, erhält man in üblicher Art und Weise entweder durch Reduktion des Carbonsäurealkylesters I (R²=CO₂R¹²) (L.I. Zakharkin, I.M. Khorlina, Tetrahedron Lett. p. 619, 1962, C. Szantay, L. Toke, P. Kolonits, J. Org. Chem. 31, p. 1447 (1966), R. Kanazawa, T. Tokoroyama, Synthesis 1976, p. 526) oder des Carbonsäurehalogenids (Y. Watanabe et al, Bull. Chem. Soc. Jpn 44, 2569 (1971), D.G. Smith, D.J.H. Smith, J.C.S. Chem. Commun. 459, 1975), R. Grewe, H. Buttner, Ber. Deutsch. Chem. Ges. 91, p. 2452 (1958). Jerry March, Advanced Organic Chemistry, Third Edition, John Wiley & Sons 1985, p. 1048 bis 1120).
   Verbindungen der Formel I, in der R² COYR⁵ bedeutet, erhält man beispielsweise durch Umsetzung einer Carbonsäure I (R² =COOH) mit einem Alkohol oder Thiol XIV in Gegenwart eines wasserentziehenden Mittels, z.B. Propanphosphonsäureanhydrid (PPA) oder Dicyclohexylcarbodiimid (DCC) bei einer Temperatur zwischen -20 und 70°C, vorzugsweise zwischen 0 und 60°C. Die Edukte werden vorteilhaft in etwa stöchiometrischer Menge umgesetzt, vorzugsweise in Gegenwart eines inerten Lösungsmittels wie Tetrahydrofuran, Dichlormethan, Toluol oder Ethylacetat. 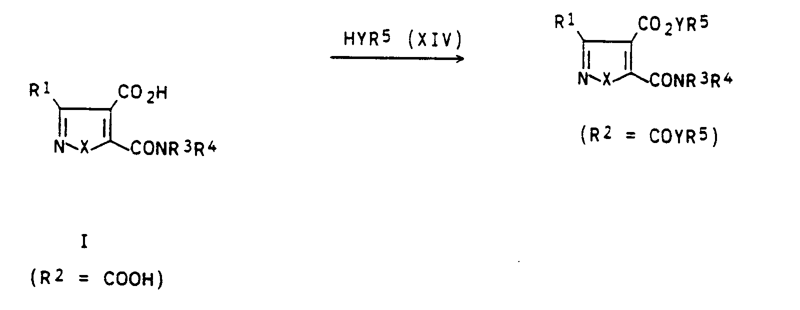 Ein weiteres Verfahren zur Herstellung von Verbindungen der Formel I, in der R² COYR⁵ oder CONR⁶R⁷ bedeutet, beruht darauf, daß man ein Säurehalogenid der Formel I (R²=COHal) in an sich bekannter Weise mit einem Alkohol oder Thiol XIV oder einem Amin der Formel Vb umsetzt. Dazu geht man zweckmäßigerweise so vor, daß man das Säurehalogenid I in einem inerten organischen Lösungsmittel vorlegt, eine Hilfsbase zutropft und anschließend den Alkohol XIV, das Thiol XIV oder das Amin Vb- vorzugsweise ebenfalls gelöst in einem inerten organischen Lösungsmittel - zutropft. Die Reaktion ist im allgemeinen nach 1 bis 12 Stunden beendet. Das Gemisch kann dann wie üblich aufgearbeitet werden, beispielsweise durch Hydrolyse mit Wasser und Extraktion des Endproduktes mit einem organischen Lösungsmittel.
   Als Lösungsmittel kommen Ether wie Diethylether, Methyl-tert.-butylether, Tetrahydrofuran und Dioxan, Halogenkohlenwasserstoffe wie Dichlormethan, Chloroform, 1,2-Dichlorethan und Chlorbenzol oder Aromaten wie Benzol, Toluol und Xylole in Betracht.
   Die Reaktionstemperatur kann zwischen -10 und 50°C, vorzugsweise 0 und 30°C, betragen.
   Als Hilfsbase verwendet man vorzugsweise tertiäre Amine wie Pyridin, N,N-Dimethylanilin oder Triethylamin.
   Die für die Verfahren 5 und 6 benötigten Amine Vb sind bekannt oder lassen sich nach bekannten Verfahren herstellen. Die Alkohole und Thiole HYR⁵ XIV sind teilweise bekannt. Bedeutet R⁵ eine durch einen Rest -CR¹⁰=N-R¹¹ substituierte C₁-C₆-Alkylgruppe, so lassen sich diese Alkohole und Thiole nach einem der folgenden bekannten Verfahren herstellen (beispielhaft für Y=O und R⁵=-CH₂CH=NOC₂H₅) gezeigt): 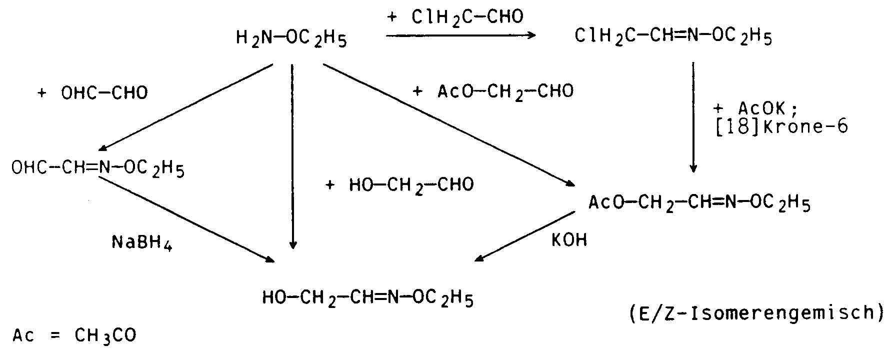 Nach den genannten Verfahren wurden z.B. die folgenden Alkohole hergestellt:
   HO-CH₂-CH=N-OCH₃, HO-CH₂-C(CH₃)=N-OCH₃, HO-CH₂-CH=N-OCH₂-CH=CHCl, HO-CH₂-CH=N-OCH₂-C₆H₅, HO-CH₂-C(CH₃)=N-OC₂H₅, HO-CH₂-CH=N-OCH₂-CH=CH₂, HO-CH₂-C(CH₃)=N-OCH₂-C₆H₅, HO-CH₂-C(CH₃)=N-OCH₂CH=CH₂.
7. Ein erfindungsgemäßes Vefahren zur Herstellung von Verbindungen der Formel I, in der R⁵ für C₁-C₆-Alkyl, daß durch einen Rest -CR¹⁰=N-R¹¹ substituiert ist, steht, besteht darin, daß man eine Isoxazol- bzw. Isothiazol-4-carbonsäure der Formel I in einem aprotisch polaren organischen Lösungsmittel vorlegt, diese mit einer Base in das Salz der Carbonsäure überführt und anschließend mit etwa einem Äquivalent eines substituierten Alkylchlorids XV (mit A = verzweigtes oder unverzweigtes C₁-C₆-Alkyl) umsetzt. Die Reaktion ist im allgemeinen nach 4 bis 20 Stunden beendet und kann wie üblich durch Zugabe von Wasser und Extraktion des Produktes mit einem organischen Lösungsmittel aufgearbeitet werden. Die Reaktionstemperatur kann zwischen 0 und 100°C vorzugsweise 20 bis 60°C variieren. Als Lösungsmittel kommt insbesondere Dimethylsulfoxid in Betracht.
   Als Basen finden Carbonate und Alkoholate der Alkali- oder Erdalkalimetalle insbesondere Kaliumcarbonat und Kalium-tert.butylat Verwendung. 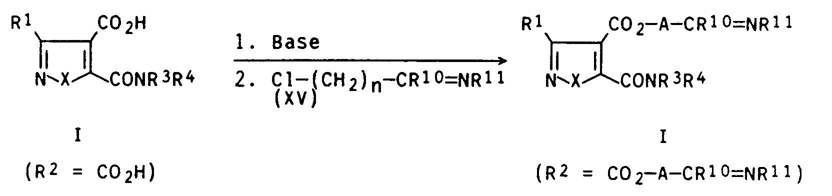
8. Verbindungen der Formel I, in der R² für COOR⁵ steht, wobei R⁵ ein salzbildendes Kation wie z.B. Alkalimetall, Erdalkalimetall, Ammonium bedeutet, werden durch Umsetzung einer substituierten Isoxazol- oder Isothiazol-4-carbonsäure I mit einem Äquivalent des salzbildenden Kations erhalten. Handelt es sich dabei um ein anorganisches Kation wie z.B. Natrium, Kalium oder Calcium, löst bzw. suspendiert man zweckmäßigerweise die Säure I in Wasser oder einem niederen Alkohol und gibt ein Äquivalent des salzbildenden Kations zu. Das salzbildende Kation kann z.B. in Form seines Hydroxids, Carbonats oder Bicarbonats, vorzugsweise in Form seines Hydroxids, eingesetzt werden. Die Reaktion ist im allgemeinen nach wenigen Minuten beendet und kann wie üblich z.B. durch Ausfällen und Absaugen oder durch Einengen der Lösung aufgearbeitet werden. Zur Herstellung von Ammoniumsalzen löst bzw. suspendiert man die Säure I in einem organischen Lösungsmittel wie z.B. Diethylether, Tetrahydrofuran oder Dioxan und behandelt die Mischung mit einem Äquivalent Ammoniak, einem Amin oder einem Tetraalkylammoniumhydroxid.
   Unter den Aminen, welche eingesetzt werden können, sollen die folgenden erwähnt werden: Methylamin, Ethylamin, n-Propylamin, Isopropylamin, n-Butylamin, Isobutylamin, sek.-Butylamin, n-Amylamin, Isoamylamin, Hexylamin, Heptylamin, Octylamin, Nonylamin, Decylamin, Undecylamin, Dodecylamin, Tridecylamin, Tetradecylamin, Pentadecylamin, Hexadecylamin, Heptadecylamin, Octodecylamin, Methylethylamin, Methylisopropylamin, Methylhexylamin, Ethylbutylamin, Ethylheptylamin, Ethyloctylamin, Hexylheptylamin, Hexyloctylamin, Dimethylamin, Diethylamin, Di-n-propylamin, Diisopropylamin, Di-n-amylamin, Diisoamylamin, Dihexylamin, Diheptylamin, Dioctylamin, Trimethylamin, Triethylamin, Tri-n-propylamin, Triisopropylamin, Tri-n-butylamin, Triisobutylamin, Tri-sek.-butylamin, Tri-n-amylamin, Ethanolamin, n-Propanolamin, Isopropanolamin, Diethanolamin, N,N-Diethylethanol-amin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Talgamin, Cyclopentylamin, Cyclohexylamin, Dicyclohexylamin, Piperidin, Morpholin und Pyrrolidin.
   Bei den Tetraalkylammoniumhydroxiden können z.B. Tetramethyl-, Tetraethyl-oder Trimethylbenzylammoniumhydroxid eingesetzt werden. In der Regel fällt das Ammoniumsalz oder organische Ammoniumsalz aus der Lösung aus und kann nach üblichen Methoden isoliert werden. Alternativ kann das Salz auch durch Einengen des Lösungsmittels erhalten werden.
9. Verbindungen der Formel I, in der R² 4,5-Dihydrooxazol-2-yl bedeutet, können durch an sich bekannte Umsetzung von Carbonsäureamiden der Formel I, wobei R² COOH oder COOR12 bedeutet, mit einem Aminoalkohol der Formel XVI erhalten werden (vgl. Theodora W. Greene, Protective Groups in Org. Synthesis, John Wiley & Sons, p. 185 (1981)). 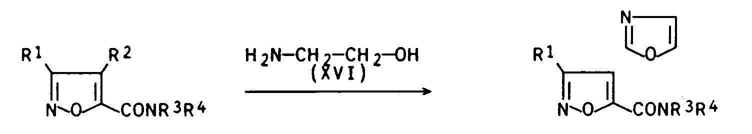 Die Reaktion wird so durchgeführt, daß man die Verbindungen bei 0 bis 180°C, vorzugsweise bei Rückflußtemperatur des verwendeten Gemisches mit einem Aminoalkohol XVI, gegebenenfalls in Gegenwart eines inerten Lösungsmittels, umsetzt. Ester oder Carbonsäure und Aminoalkohol XVI werden dabei im Verhältnis 1:1 bis 1:2,5, vorzugweise 1:1 bis 1:1,5 eingesetzt. Im allgemeinen arbeitet man bei Atmosphärendruck oder unter dem Eigendruck des jeweiligen Lösungsmittels.
   Als Lösungsmittel verwendet man zweckmäßigerweise Halogenkohlenwasserstoffe wie Chlorbenzol und 1,2-Dichlorbenzol, Ether, z.B. Methyltert.-butylether, 1,2-Dimethoxyethan, Diethylenglykol-dimethylether, Tetrahydrofuran und Dioxan, Alkohole wie Methanol, Ethanol, Propanol oder Ethylenglykol, dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten, z.B. Benzol, Toluol und Xylol. Die Konzentration der Edukte im Lösungsmittel beträgt im allgemeinen 0,1 bis 5 mol/l, bevorzugt 0,2 bis 2 mol/l.
   Die Umsetzung ist im allgemeinen nach 14 Stunden beendet; die Carbonsäureamide I, (mit R2=4,5-Dihydrooxazol-2-yl) werden dann gegebenenfalls durch Zugabe von Wasser ausgefällt, abgesaugt oder mit einem organischen Lösungsmittel extrahiert und nach üblichen Standardmethoden wie Umkristallisation oder Chromatographie gereinigt.

Die Substituenten in den erfindungsgemäßen Verbindungen I haben beispielsweise die folgende Bedeutung:
X
Sauerstoff oder Schwefel;
R¹
Halogen wie Fluor, Chlor, Brom, Jod, insbesondere Chlor und Brom;
C₁-C₄-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio,
C₁-C₄-Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy,
C₁-C₄-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluor-ethylthio, 2,2,2-Trichlorethylthio und Pentafluorethythio, insbesondere Trifluormethylthio und Pentafluorethylthio,
Cyano, Formyl,
C₂-C₄-Alkanoyl wie Ethanoyl, Propanoyl, 2-Methylpropanoyl, Butanoyl, insbesondere Ethanoyl (Acetyl) und 2-Methylpropanoyl,
C₁-C₄-Alkoxycarbonyl wie Methoxycarbonyl, Ethoxycarbonyl, Propoxycarbonyl, 1-Methylethoxycarbonyl, Butoxycarbonyl und 1,1-Dimethylethoxycarbonyl, insbesondere Methoxycarbonyl,
C₁-C₄-Alkylaminocarbonyl wie Methylaminocarbonyl, Ethylaminocarbonyl, Propylaminocarbonyl, 1-Methylethylaminocarbonyl, Butylaminocarbonyl, 1,1-Dimethylethylaminocarbonyl, insbesondere Methylaminocarbonyl, Ethylaminocarbonyl, 1-Methylethylaminocarbonyl und 1,1-Dimethylethylaminocarbonyl,
Di-(C₁-C₃-)alkylaminocarbonyl wie Dimethylaminocarbonyl, Diethylaminocarbonyl, Dipropylaminocarbonyl und Di-(1-Methylethyl)aminocarbonyl, insbesondere Dimethylaminocarbonyl und Methylethylaminocarbonyl,
Cyclopropylaminocarbonyl,
Methylsulfonyl, Trifluormethylsulfonyl;
Phenoxy oder Phenylthio, welches ein- bis dreimal durch C₁-C₄-Alkyl wie Methyl, Ethyl, Propyl, 1-Methylethyl, Butyl, 1-Methylpropyl, 2-Methylpropyl, 1,1-Dimethylethyl, insbesondere Methyl,
C₁-C₄-Halogenalkyl wie Difluormethyl, Trifluormethyl, Chlordifluormethyl, Dichlorfluormethyl, 1-Fluorethyl, 2-Fluorethyl, 2,2-Difluorethyl, 1,1,2,2-Tetrafluorethyl, 2,2,2-Trifluorethyl, 2-Chlor-1,1,2-trifluorethyl und Pentafluorethyl, insbesondere Trifluormethyl,
C₁-C₄-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,
C₁-C₄-Halogenalkoxy wie vorstehend genannt, insbesondere Trifluormethoxy,
C₁-C₄-Alkylthio wie vorstehend genannt, insbesondere Methylthio,
C₁-C₄-Halogenalkylthio wie vorstehend genannt, insbesondere Trifluormethylthio,
Halogen, Cyano oder Nitro substituiert sein kann;
R2
die Formylgruppe; die Carbonsäurefluorid-, -chlorid-, -bromidgruppe,
die 4,5-Dihydrooxazol-2-ylgruppe;
ein Rest COYR⁵ mit
R5
Wasserstoff;
C₁-C₆-Alkyl, bevorzugt C₁-C₄-Alkyl wie Methyl, Ethyl, 1-Methylethyl, 1,1-Dimethylethyl, welches ein bis fünf Halogenatome wie Fluor, Chlor, Brom oder Jod, insbesondere Fluor und Chlor, und/oder bis zu drei Hydroxy- und/oder C₁-C₄-Alkoxygruppen wie Methoxy, Ethoxy, n-Propoxy, 1-Methylethoxy, n-Butoxy unt 1,1-Dimethylethoxy, und/oder einen der folgenden Reste tragen:
- Cyano,
- C₁-C₄-Alkoxy-C₂-C₄-alkoxy, insbesondere Methoxy-ethoxy, Ethoxy-ethoxy und Propoxy-ethoxy,
- C₁-C₃-Alkylthio, insbesondere Methylthio und Ethylthio,
- C₁-C₃-Alkylamino, wie Methylamino, Ethylamino und 1-Methylethylamino,
- Di-(C₁-C₃)-alkylamino, wie Dimethylamino, Diethylamino, Dipropylamino, Di-(1-methylethyl)amino und Methylethylamino,
- C₃-C₆-Cycloalkylamino oder Di-(C₃-C₆)-Cycloalkylamino wie Cyclopropylamino oder Dicyclopropylamino
- Trimethylsilyl,
- C₁-C₃-Alkylsulfinyl wie Methylsulfinyl, 1-Methylethylsulfinyl und n-Propylsulfinyl, insbesondere Methylsulfinyl,
- C₁-C₃-Alkylsulfonyl wie Methylsulfonyl, Ethylsulfonyl, 1-Methylethylsulfonyl,
- Carboxyl
- C₁-C₃-Alkoxycarbonyl wie Methoxycarbonyl und Ethoxycarbonyl,
- C₁-C₃-Alkoxycarbonyl-C₁-C₃-alkoxy wie Methoxycarbonylethoxy,
- C₁-C₃-Alkoxycarbonyl-C₁-C₃-alkoxy-C₁-C₃-alkoxycarbonyl wie Methoxycarbonylethoxymethoxycarbonyl,
- Di-(C₁-C₃)-alkylaminocarbonyl wie Dimethylaminocarbonyl, Methylethylaminocarbonyl und Di-(1-Methylethyl)-aminocarbonyl,
- Di-(C₁-C₃)-alkoxyphosphonyl wie Dimethoxyphosphonyl und, Diethoxyphosphonyl,
- C₁-C₆-Alkaniminoxy wie 2-Propaniminoxy oder C₅-C₆-Cycloalkaniminoxy wie Cyclopentaniminoxy und Cyclohexaniminoxy,
- N-Phthalimido, N-Succinimido, Benzyloxy oder Benzoyl, wobei diese cyclischen Reste ein bis drei der folgenden Gruppen tragen können: Halogen wie vorstehend genannt, insbesondere Fluor und Chlor, C₁-C₃-Alkyl wie Methyl, Ethyl und 1-Methylethyl, insbesondere Methyl, Ethyl und 1-Methylethyl, oder C₁-C₃-Alkoxy wie Methoxy, Ethoxy und 1-Methylethoxy, insbesondere Methoxy,
- einen 5- oder 6-gliedrigen gesättigten heterocyclischen Rest oder einen 5- oder 6-gliedrigen heteroaromatischen Rest mit jeweils 1 bis 3 Heteroatomen, ausgewählt aus der Gruppe Sauerstoff, Schwefel und Stickstoff, wobei zwei Sauerstoff- oder Schwefelatome oder ein Sauerstoff und ein Schwefelatom nicht direkt benachbart sind, insbesondere Tetrahydrofuran-2-yl, Tetrahydrofuran-3-yl, Tetrahydrothien-2-yl, Tetrahydrothien-3-yl, Tetrahydropyran-2-yl, Tetrahydropyran-3-yl, Tetrahydropyran-4-yl, Pyrrolidin-2-yl, Pyrrolidin-3-yl, Furan-2-yl, Furan-3-yl, Thien-2-yl, Thien-3-yl, Pyrrol-2-yl, Pyrrol-3-yl, Isoxazol-3-yl, Isoxazol-4-yl, Isoxazol-5-yl, Isothiazo-3-yl, Isothiazo-4-yl, Isothiazol-5-yl, Oxazol-2-yl, Oxazol-4-yl, Oxazol-5-yl, Thiazol-2-yl, Thiazol-4-yl, Thiazol-5-yl, Imidazol-2-yl, Imidazol-4-yl, Imidazol-5-yl, Pyrazol-1-yl, Pyrazol-3-yl, Pyrazol-4-yl, Pyrazol-5-yl, 1,2,3-Triazol-1-yl, 1,2,3-Triazol-4-yl, 1,2,3-Triazol-5-yl, 1,2,4-Triazol-1-yl, 1,2,4-Triazol-3-yl, 1,2,4-Triazol-5-yl, 1,2,3-Thiadiazol-4-yl, 1,2,3-Thiadiazol-5-yl, 1,2,4-Thiadiazol-3-yl, 1,2,4-Thiadiazol-5-yl, 1,2,5-Thiadiazol-3-yl, 1,2,5-Thiadiazol-4-yl, 1,3,4-Thiadiazol-2-yl, 1,3,4-Thiadiazol-5-yl, 1,2,3-Oxadiazol-3-yl, 1,2,3-Oxadiazol-5-yl, 1,2,4-Oxadiazol-3-yl, 1,2,4-Oxadiazol-5-yl, 1,2,5-Oxadiazol-3-yl, 1,2,5-Oxadiazol-4-yl, 1,3,4-Oxadiazol-2-yl, 1,3,4-Oxadiazol-5-yl, Pyrid-2-yl, Pyrid-3-yl, Pyrid-4-yl, Pyrimid-2-yl, Pyrimid-4-yl und Pyrimid-5-yl, wobei die Heterocyclen noch einen oder zwei der folgenden Substituenten tragen können: Halogen wie Fluor, Chlor, Brom und Jod, insbesondere Chlor und Brom, C₁-C₃-Alkyl wie Methyl, Ethyl, n-Propyl und 1-Methylethyl, C₁-C₃-Alkoxy wie Methoxy, Ethoxy, n-Propoxy und 1-Methylethoxy und/oder C₁-C₃-Alkoxycarbonyl wie Methoxycarbonyl, Ethoxycarbonyl und 1-Methylethoxycarbonyl;
- die Phenylgruppe, die noch einen bis drei der folgenden Substituenten tragen kann: Halogen wie Fluor, Chlor, Brom und Iod, insbesondere Fluor und Chlor, Nitro, Cyano, C₁-C₃-Alkyl wie Methyl, Ethyl oder 1-Methylethyl, partiell oder vollständig halogeniertes C₁-C₃-Alkyl wie Trifluormethyl, 1,1,2,2-Tetrafluorethyl und Trichlormethyl, C₁-C₃-Alkoxy wie Methoxy und 1-Methylethoxy und/oder partiell oder vollständig halogeniertes C₁-C₃-Alkoxy, insbesondere Trifluormethoxy oder C₁-C₂-Alkoxycarbonyl, insbesondere Methoxycarbonyl;
- einen Rest -CR¹⁰=N-R¹¹ mit
R¹⁰
Wasserstoff oder verzweigtes oder unverzweigtes C₁-C₆-Alkyl, insbesondere C₁-C₄-Alkyl wie Methyl, Ethyl, n-Propyl, 1-Methylethyl, n-Butyl und 1,1-Dimethylethyl,
R¹¹
C₁-C₆-Alkoxy, C₃-C₆-Alkenyloxy oder C₃-C₆-Alkinyloxy, insbesondere C₁-C₄-Alkoxy wie Methoxy, Ethoxy, n-Propoxy, 1-Methylethoxy, n-Butoxy und 1,1-Dimethylethoxy, sowie Prop-2-enyloxy, But-2-enyloxy. Prop-2-inyloxy und But-2-inyloxy, wobei diese Substituenten noch ein bis drei Halogenatome wie Fluor, Chlor, Brom und Iod, insbesondere Fluor und Chlor, und/oder einen Phenylrest, der unsubstituiert oder ein- bis dreifach durch Halogen wie vorstehend genannt, Nitro, Cyano, C₁-C₃-Alkyl wie Methyl, Ethyl und n-Propyl und/oder C₁-C₃-Alkoxy wie Methoxy, Ethoxy, n-Propoxy und 1-Methylethoxy substituiert sein kann, tragen können;
Phenoxy, das noch einen bis drei der folgenden Substituenten tragen kann: Nitro, Cyano, Halogen wie vorstehend genannt, C₁-C₃-Alkyl wie vorstehend genannt und/oder C₁-C₃-Alkoxy wie vorstehend genannt; verzweigtes oder unverzweigtes C₁-C₆-Alkylamino, insbesondere Methylamino, Ethylamino, Di-(C₁-C₆)-alkylamino, insbesondere Dimethylamino, Methylethylamino oder Phenylamino, wobei der Aromat zusätzlich ein- bis dreifach durch Nitro, Cyano, Halogen wie vorstehend genannt, C₁-C₃-Alkyl wie vorstehend genannt und/oder C₁-C₃-Alkoxy wie vorstehend genannt, substituiert sein kann;
R⁵ ferner:
C₃-C₈-Cycloalkyl, bevorzugt C₃-C₆-Cycloalkyl wie Cyclopropyl, Cyclopentyl und Cyclohexyl;
C₃-C₆-Alkenyl, bevorzugt C₃-C₄-Alkenyl wie 2-Propenyl und 2-Butenyl, C₅-C₆-Cycloalkenyl wie 2-Cyclopentenyl und 2-Cyclohexenyl, C₃-C₆-Alkinyl, bevorzugt C₃-C₄-Alkinyl wie 2-Propinyl, 2-Butinyl und 3-Butinyl, wobei 3 letztgenannten Gruppen einen der folgenden Reste tragen können: Hydroxy, Halogen wie Fluor, Chlor, Brom und Jod, C₁-C₄-Alkoxy wie Methoxy und 1,1-Dimethylethoxy oder Phenyl, welches seinerseits eine bis drei der folgenden Gruppen tragen kann: Halogen wie Fluor, Chlor oder Brom, Nitro, Cyano, C₁-C₄-Alkyl wie Methyl oder Ethyl, C₁-C₄-Halogenalkyl wie Fluormethyl, Trifluormethyl, Chlordifluormethyl, Pentafluorethyl und 2-Chlor-1,1,2-trifluorethyl oder C₁-C₄-Alkoxy wie Methoxy, 1-Methyethoxy, 1,1-Dimethylethoxy;
Phenyl, das eine bis drei der folgenden Gruppe tragen kann:
- Halogen wie Fluor, Chlor, Brom und Jod, insbesondere Fluor und Chlor, Nitro, Cyano, C₁-C₄-Alkyl wie Methyl, Ethyl und 1,1-Dimethylethyl, partiell oder vollständig halogeniertes C₁-C₄-Alkyl wie Trifluormethyl, 1,1,2,2-Tetrafluorethyl und Trichlormethyl, C₁-C₄-Alkoxy wie Methoxy, Ethoxy und 1-Methylethoxy, partiell oder vollständig halogeniertes C₁-C₄-Alkoxy wie Trifluormethoxy, Chlordifluormethoxy, 1-Fluorethoxy, Pentafluorethoxy und 2-Chlor-1,1,2-trifluorethoxy oder C₁-C₄-Alkoxycarbonyl wie Methoxycarbonyl, n-Propoxycarbonyl und 1,1-Dimethylethoxycarbonyl,
ein 5- oder 6-gliedriger gesättigter heterocyclischer Rest oder ein 5- oder 6-gliedriger heteroaromatischer Rest mit jeweils 1 bis 3 Heteroatomen, ausgewählt aus der Gruppe Sauerstoff, Schwefel und Stickstoff, wobei zwei Sauerstoff- oder Schwefelatome oder ein Sauerstoff- und ein Schwefelatom nicht direkt benachbart sein können, wie vorstehend genannt, insbesondere 2-Tetrahydrofuranyl, 3-Tetrahydrothienyl, 4-Tetrahydropyranyl, 2-Furanyl, 2-Thienyl, 4-Isoxazolyl, 5-Isothiazolyl, 2-Oxazolyl, 4-Thiazolyl, 2-Imidazolyl, 2-Pyrrolyl, 3-Pyrazolyl und 4-Pyridyl, wobei die Heterocyclen noch einen oder zwei der folgenden Substituenten tragen können: Halogen wie vorstehend genannt, C₁-C₃-Alkyl wie vorstehend genannt, C₁-C₃-Alkoxy wie vorstehend genannt und/oder C₁-C₃-Alkoxycarbonyl wie vorstehend genannt;
ein Benzotriazolrest;
N-Phthalimido, Tetrahydrophthalimido, N-Succinimido oder Maleinimido;
die 2,2-Dimethyl-1,3-dioxolan-4-ylmethyl- oder die 1,3-Di-oxolan-2-on-4-ylmethylgruppe;
im Falle Y=O: ein Äquivalent eines Kations aus der Gruppe der Alkali- und Erdalkalimetalle wie Natrium, Kalium und Calcium, Mangan, Kupfer, Eisen, Ammonium und mit bis zu 4 C₁-C₃-Alkylgruppen substituiertes Ammonium wie Tetramethylammonium;
ein Rest -N=CR⁸R⁹ mit
R⁸, R⁹
Wasserstoff
C₁-C₄-Alkyl oder partiell oder vollständig halogeniertes C₁-C₄-Alkyl wie Methyl, Ethyl, 1-Methylethyl, tert.-Butyl, Chlormethyl, Fluormethyl, Trifluormethyl, Trichlormethyl und 1,1,2,2-Tetrafluorethyl, wobei die Alkyl- oder Halogenalkylgruppe noch einen der folgenden Reste tragen kann:
- C₁-C₃-Alkoxy wie vorstehend genannt, insbesondere Methoxy,
- Phenyl, das zusätzlich ein- bis dreimal durch Nitro, Cyano, Halogen wie vorstehend genannt, insbesondere Fluor und Chlor, C₁-C₃-Alkyl wie vorstehend genannt, insbesondere Methyl, Ethyl und 1-Methylethyl, partiell oder vollständig halogeniertes C₁-C₃-Alkyl wie vorstehend genannt, insbesondere Trifluormethyl, C₁-C₃-Alkoxy wie vorstehend genannt, insbesondere Methoxy und/oder partiell oder vollständig halogeniertes C₁-C₃-Alkoxy wie vorstehend genannt, insbesondere Trifluormethoxy, substituiert sein kann;
C₃-C₆-Cycloalkyl wie Cyclopropyl, Cyclopentyl und Cyclohexyl;
C₁-C₄-Alkoxy wie vorstehend genannt, insbesondere Methoxy und Ethoxy;
Furanyl oder Phenyl, das zusätzlich ein- bis dreimal durch Nitro, Cyano, Halogen wie vorstehend genannt, insbesondere Fluor und Chlor, C₁-C₃-Alkyl wie vorstehend genannt, insbesondere Methyl, Ethyl und 1-Methylethyl, partiell oder vollständig halogeniertes C₁-C₃-Alkyl wie vorstehend genannt, insbesondere Trifluormethyl, C₁-C₃-Alkoxy wie vorstehend genannt, insbesondere Methoxy und/oder partiell oder vollständig halogeniertes C₁-C₃-Alkoxy wie vorstehend genannt, insbesondere Trifluormethoxy, substituiert sein kann;
R⁸, R⁹
gemeinsam eine Methylenkette mit 4 - 7, bevorzugt 4 - 5 Gliedern;
oder R⁵ ein Rest -W-Z bedeutet, in dem
W
eine Ethylen-, n-Propylen oder n-Butylenkette, eine Ethoxyethylen-, But-2-enylen- oder But-2-inylenkette darstellt und
Z
für einen in ω-Stellung an W gebundenen Molekülteil, der den gleichen Molekülteil darstellt, der in α-Stellung von W mit W verknüpft ist, beispielsweise 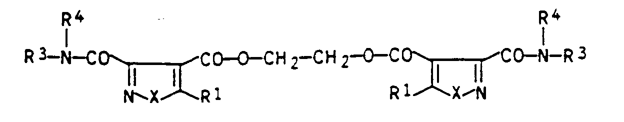
R³
Wasserstoff;
C₁-C₆-Alkyl, bevorzugt C₁-C₄-Alkyl wie Methyl, Ethyl, 1-Methylethyl und 1,1-Dimethylethyl, das einen bis drei der folgenden Substituenten tragen kann: Hydroxy, Halogen, C₁-C₄-Alkoxy wie Methoxy und 1,1-Dimethylethoxy, C₁-C₄-Alkylthio wie Methylthio und 1,1-Dimethylethylthio oder Di-(C₁-C₄)-alkylamino, bevorzugt Di-(C₁-C₂)-alkylamino wie Dimethylamino und Diethylamino;
C₃-C₈-Cycloalkyl, bevorzugt C₃-C₆-Cycloalkyl wie Cyclopropyl, Cyclopentyl und Cyclohexyl, das ein- bis dreimal durch Halogen wie Fluor, Chlor und Brom, C₁-C₄-Alkyl wie Methyl und 1,1-Dimethylethyl oder partiell oder vollständig halogeniertes C₁-C₄-Alkyl wie Fluormethyl, Trifluormethyl, Chlordifluormethyl, Pentafuorethyl und 2-Chlor-1,1,2-trifluorethyl substituiert sein kann;
R⁴
Wasserstoff, Hydroxyl;
C₁-C₄-Alkoxy wie Methoxy, Ethoxy, 1-Methylethoxy, 1,1-Dimethylethoxy;
verzweigtes oder unverzweigtes C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethybutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl und 1,1-Dimethylethyl, welches ein bis drei der folgenden Reste tragen kann: Halogen wie Fluor, Chlor und Brom, Cyano, C₁-C₄-Alkoxy wie Methoxy, Ethoxy und 1,1-Dimethylethoxy, partiell oder vollständig halogeniertes C₁-C₄-Alkoxy wie Fluormethoxy, Trichlormethoxy, 2-Chlor-1,1,2-trifluorethoxy und Pentafluorethoxy, C₁-C₄-Alkylthio wie Methylthio, Ethylthio und 1,1-Dimethylethylthio, C₁-C₄-Halogenalkylthio wie Fluormethylthio, Trichlormethylthio, 2-Chlor-1,1,2-trifluorethylthio und Pentafluorethylthio, Di-(C₁-C₄)alkylamino, insbesondere Di-(C₁-C₂)-alkylamino wie Dimethylamino und Diethylamino, C₃-C₈-Cycloalkyl, insbesondere C₃-C₆-Cycloalkyl wie Cyclopropyl, Cyclopentyl und Cyclohexyl oder Phenyl, wobei der Phenylrest seinerseits bis zu drei der folgenden Gruppen tragen kann: Halogen wie Fluor, Chlor und Brom, Cyano, Nitro, C₁-C₄-Alkyl wie Methyl, Ethyl, und 1,1-Dimethylethyl, C₁-C₄-Halogenalkyl wie Fluormethyl, Trichlormethyl, 2-Chlor-1,1,2-trifluorethyl und Pentafluorethyl, C₁-C₄-Alkoxy wie Methoxy und 1,1-Dimethylethoxy, partiell oder vollständig halogeniertes C₁-C₄-Alkoxy wie Fluormethoxy, Trichlormethoxy, 2-Chlor-1,1,2-trifluorethoxy und Pentafluorethoxy, C₁-C₄-Alkylthio wie Methylthio und 1,1-Dimethylethylthio oder C₁-C₄-Halogenalkylthio wie Fluormethylthio, Trichlormethylthio, 2-Chlor-1,1,2-trifluorethylthio und Pentafluorethylthio;
C₃-C₈-Cycloalkyl, bevorzugt C₃-C₆-Cycloalkyl, insbesondere Cyclopropyl, Cyclopentyl und Cyclohexyl, das jeweils einen bis drei der folgenden Reste tragen kann: Halogen wie Fluor, Chlor und Brom, Nitro, Cyano, C₁-C₆-Alkyl, bevorzugt C₁-C₄-Alkyl wie Methyl, Ethyl und 1,1-Diethylethyl, partiell oder vollständig halogeniertes C₁-C₆-Alkyl bevorzugt C₁-C₄-Halogenalkyl wie Fluormethyl, Trichlormethyl, 2-Chlor-1,1,2-trifluorethyl und Pentafluorethyl, C₁-C₄-Alkoxy wie Methoxy, 1,1-Dimethylethoxy oder partiell oder vollständig halogeniertes C₁-C₄-Alkoxy wie Fluormethoxy, Trichlormethoxy, 2-Chlor-1,1,2-trifluorethoxy und Pentafluorethoxy;
C₃-C₆-Alkenyl, wobei die Doppelbindung epoxidiert sein kann, oder C₃-C₆-Alkinyl, bevorzugt C₃-C₄-Alkenyl oder C₃-C₄-Alkinyl wie 2-Propenyl, 2-Butenyl, 1,1-Dimethyl-2-propenyl, 2-Propinyl, 1,1-Dimethyl-2-propinyl und 3-Butinyl, welche jeweils bis zu dreifach durch Halogen wie Fluor, Chlor oder Brom und/oder einmal durch Phenyl substituiert sein können, wobei der Phenylrest seinerseits einen bis drei der folgenden Substituenten tragen kann: Halogen, insbesondere Fluor und Chlor, Cyano, Nitro, C₁-C₄-Alkyl wie Methyl und 1,1-Dimethylethoxy, partiell oder vollständig halogeniertes C₁-C₄-Alkyl wie Fluormethyl, Trifluormethyl, Trichlormethyl, 2-Chlor-1,1,2-trifluorethyl und Pentafluorethyl, C₁-C₄-Alkoxy wie Methoxy und 1,1-Dimethylethoxy, C₁-C₄-Halogenalkoxy wie Fluormethoxy, Trifluormethoxy, Trichlormethoxy, 2-Chlor-1,1,2-trifluorethoxy und Pentafluorethoxy, C₁-C₄-Alkylthio wie Methylthio und 1,1-Dimethylethylthio, partiell oder vollständig halogeniertes C₁-C₄-Alkylthio wie Fluormethylthio, Trifluormethylthio, Trichlormethylthio, 2-Chlor-1,1,2-trifluorethylthio und Pentafluorethylthio;
Di(C₁-C₄)-alkylamino, bevorzugt Di-(C₁-C₂)-alkylamino wie Dimethylamino und Diethylamino;
ein 5- bis 6-gliedriger gesättigter oder aromatischer heterocyclischer Rest, enthaltend ein oder zwei Heteroatome, ausgewählt aus der Gruppe Sauerstoff, Schwefel und Stickstoff wie 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrazolyl, 4-Pyrazolyl, 5-Pyrazolyl, 2-Pyridyl, 3-Pyridyl und 4-Pyridyl, der einen bis drei der folgenden Substituenten tragen kann: C₁-C₄-Alkyl wie vorstehend genannt, insbesondere Methyl oder Halogen wie vorstehend genannt, insbesondere Fluor und Chlor;
Phenyl, welches eine bis vier der folgenden Grupppen tragen kann: C₁-C₄-Alkyl wie vorstehend genannt, insbesondere Methyl, Ethyl und 1-Methylethyl; partiell oder vollständig halogeniertes C₁-C₄-Alkyl wie vorstehend genannt, insbesondere Trifluormethyl und Chlordifluormethyl; C₁-C₄-Alkoxy wie vorstehend genannt, insbesondere Methoxy und Ethoxy; partiell oder vollständig halogeniertes C₁-C₄-Alkoxy wie vorstehend genannt, insbesondere Trifluormethoxy, Trichlormethoxy und Pentafluorethoxy; C₁-C₄-Alkylthio wie vorstehend genannt, insbesondere Methylthio und Ethylthio; partiell oder vollständig halogeniertes C₁-C₄-Alkylthio wie vorstehend genannt, insbesondere Difluormethylthio, Trifluormethylthio und Pentafluormethylthio, Halogen wie vorstehend genannt, insbesondere Fluor und Chlor, Cyano, Nitro, Formyl, C₂-C₄-Alkanoyl wie Ethanoyl, Propanoyl und 1-Methylethanoyl, insbesondere Ethanoyl, partiell oder vollständig halogeniertes C₂-C₄-Alkanoyl wie Trifluorethanoyl, Trichlorethanoyl, Pentafluorpropanoyl, insbesondere Trifluorethanoyl oder C₁-C₄-Alkoxycarbonyl wie Methoxycarbonyl und Ethoxycarbonyl;
Naphthyl, das ein- bis dreimal durch C₁-C₄-Alkyl wie vorstehend genannt, insbesondere Methyl und Ethyl, oder Halogen wie Fluor und Chlor substituiert sein kann;
R³ und R⁴ gemeinsam eine C₄-C₇-Methylenkette, welche durch Sauerstoff, Schwefel oder N-Methyl unterbrochen sein kann wie -(CH₂)₃-, -(CH₂)₄-, -(CH₂)₅-, -(CH₂)₆-, -CH₂-O-CH₂-, -CH₂-CH₂-O-CH₂-CH₂-, -CH₂-S-CH₂-, -CH₂-CH₂-S-CH₂-CH₂-, -CH₂-CH₂-N(CH₃)-CH₂-CH₂-, insbesondere -(CH₂)₅- und -CH₂-CH₂-O-CH₂-CH₂-;
oder den Rest der Formel -(CH₂)₃-CO-.

Verbindungen I, die im Hinblick auf ihre bestimmungsgemäße Verwendung besonders bevorzugt sind, sind in den folgenden Tabellen aufgeführt:

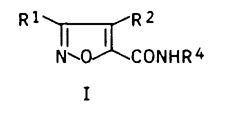

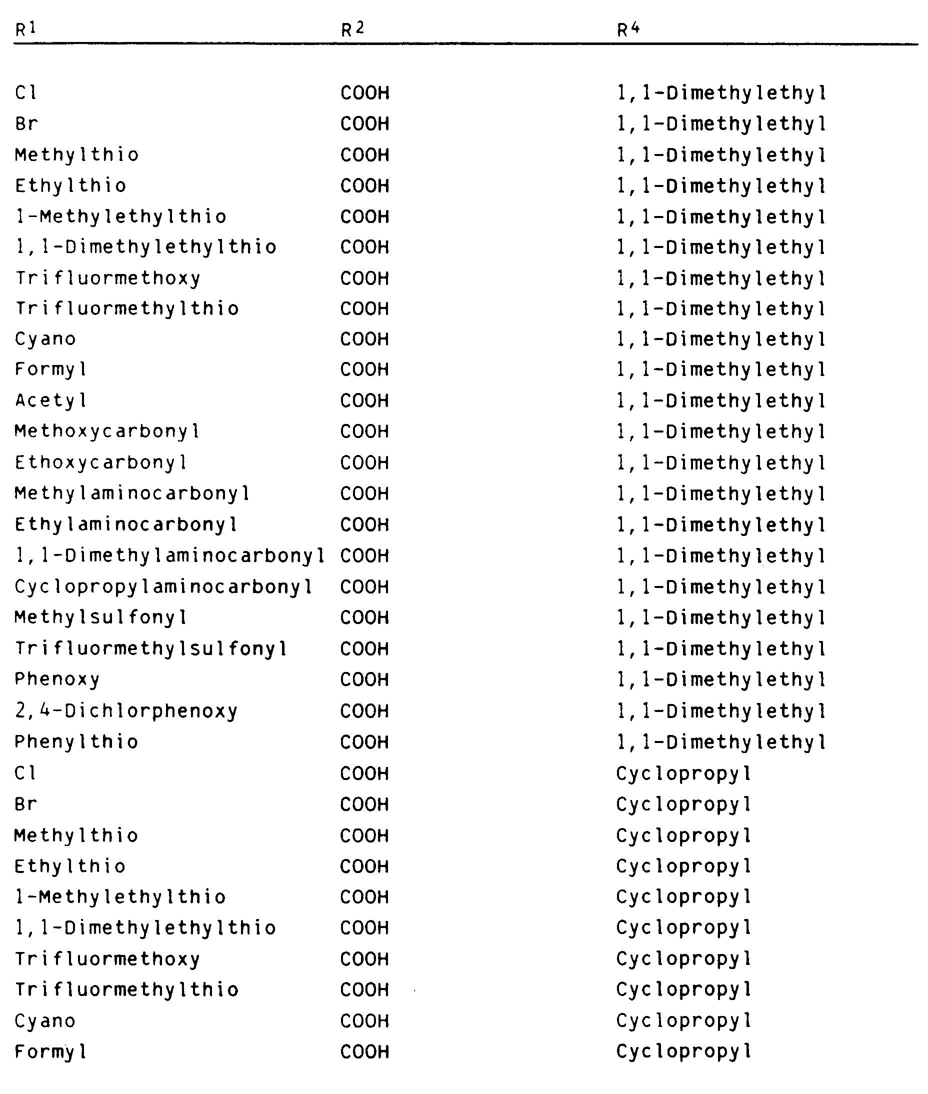

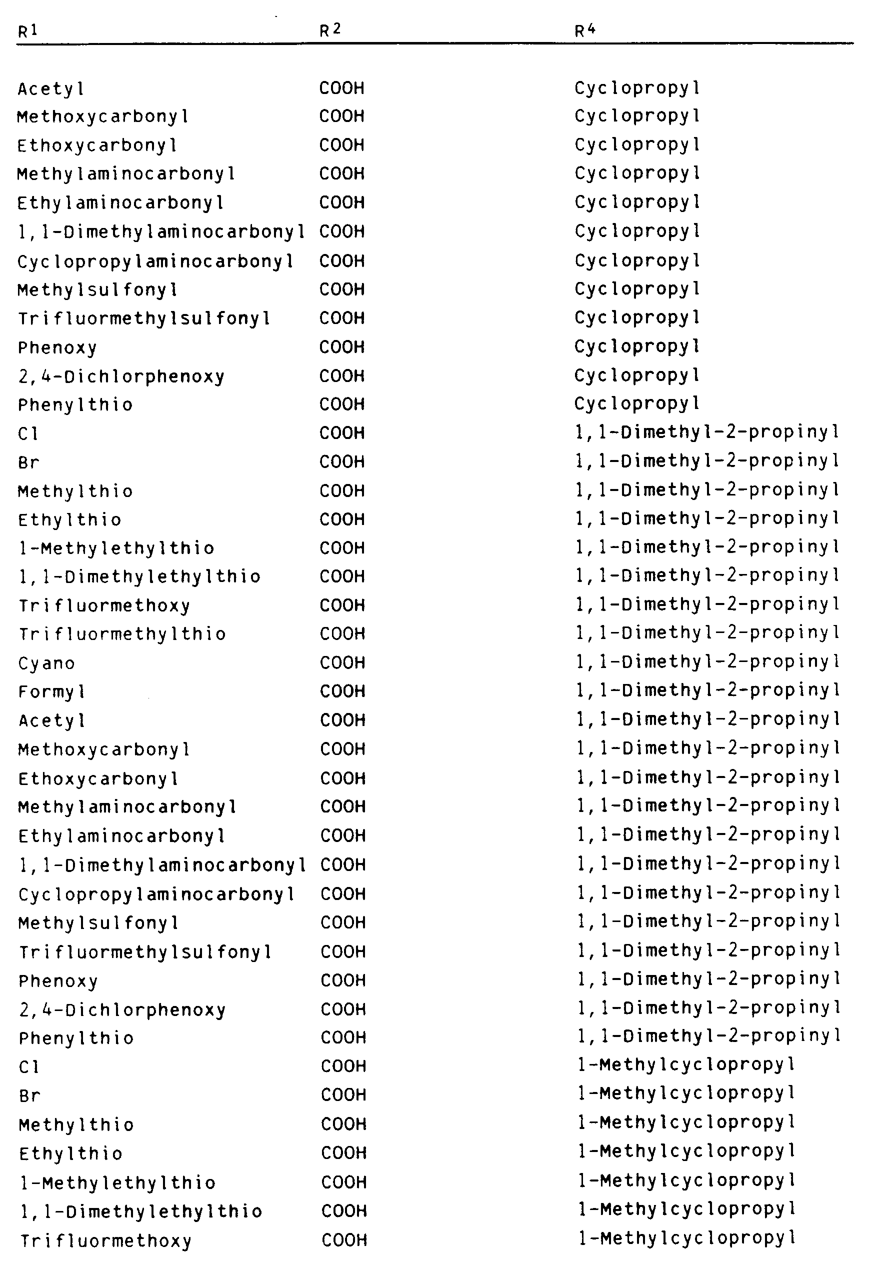

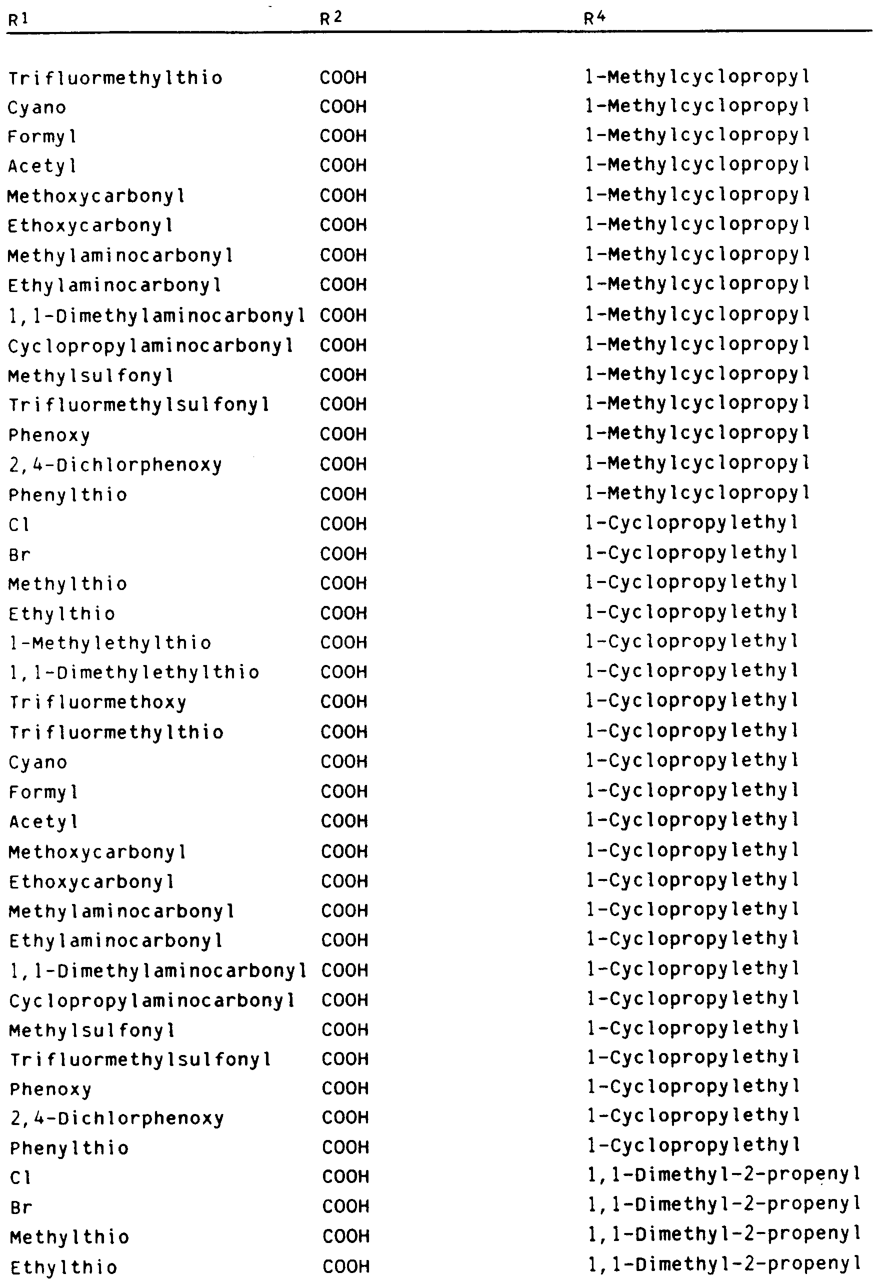

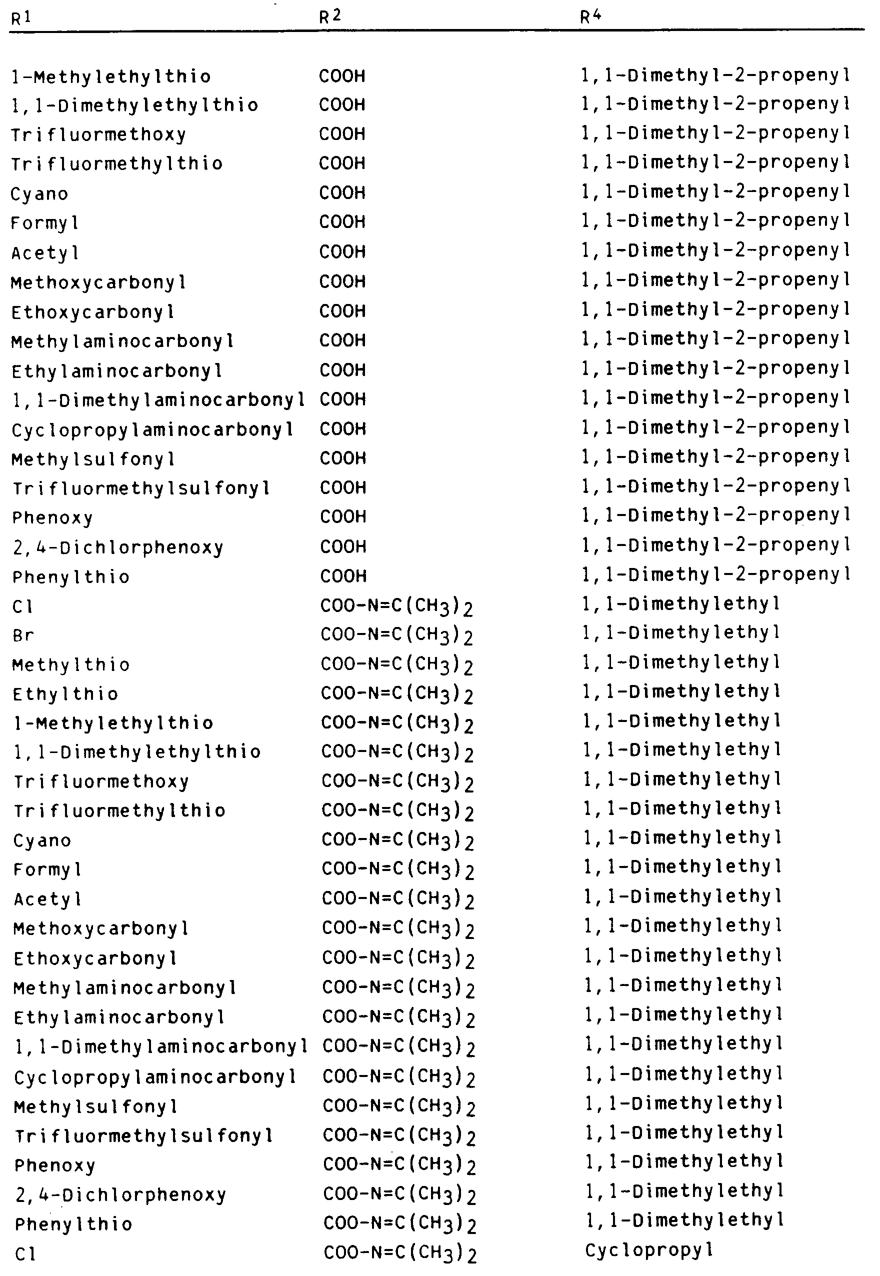

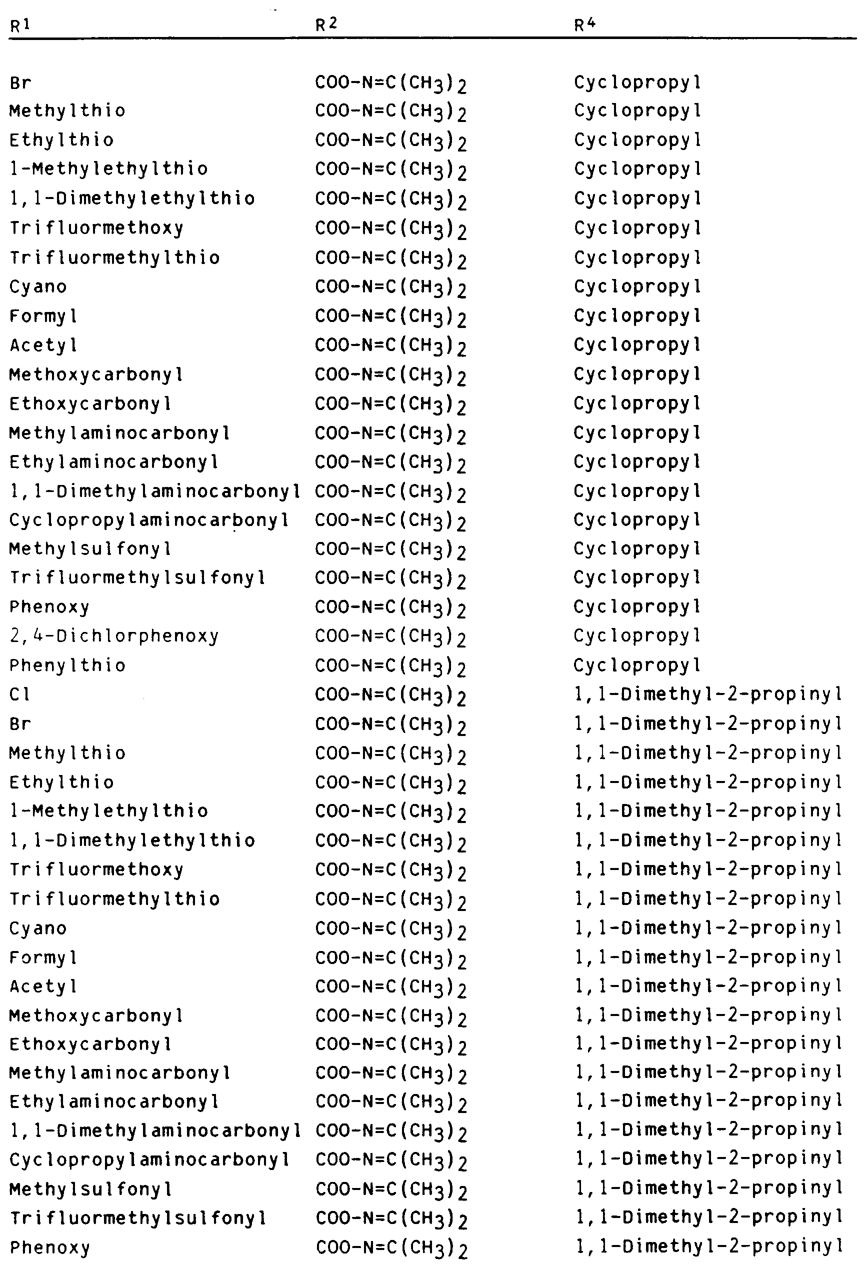

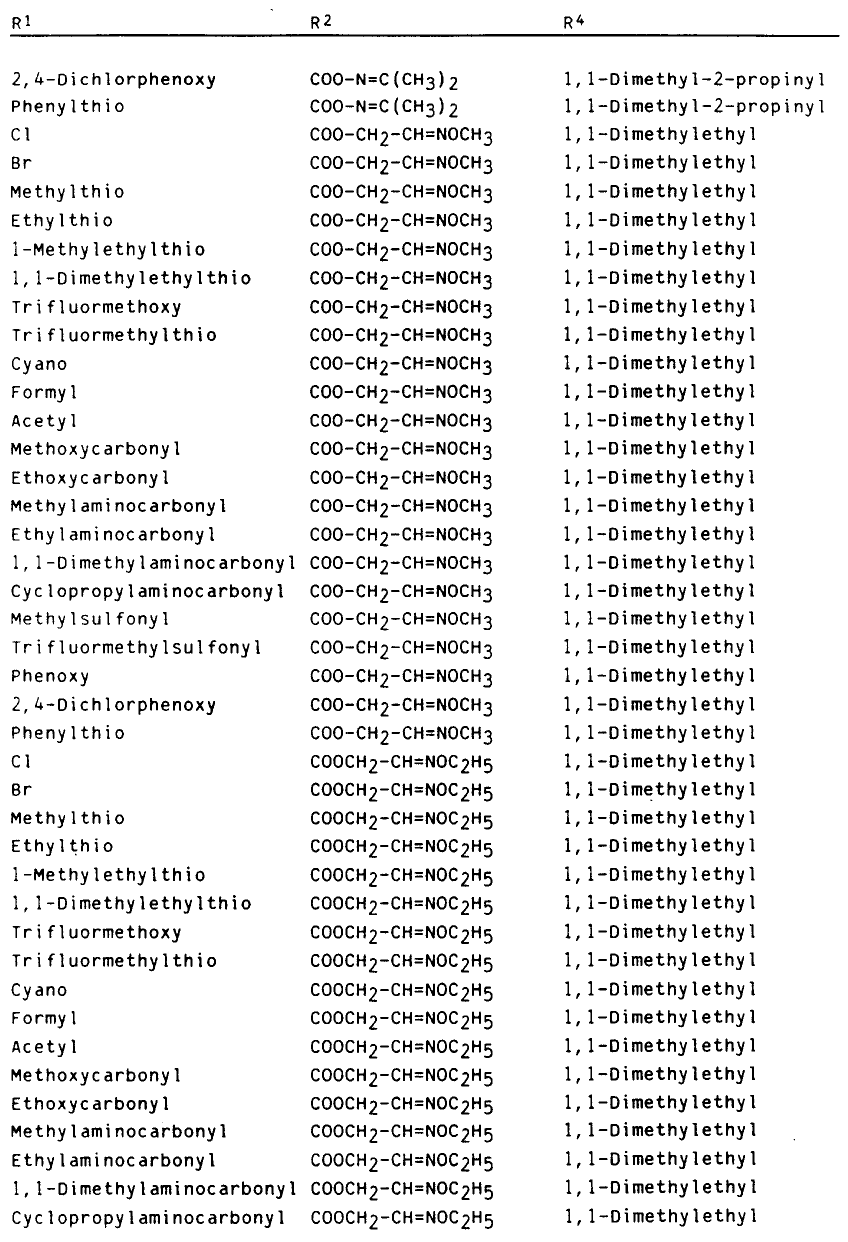

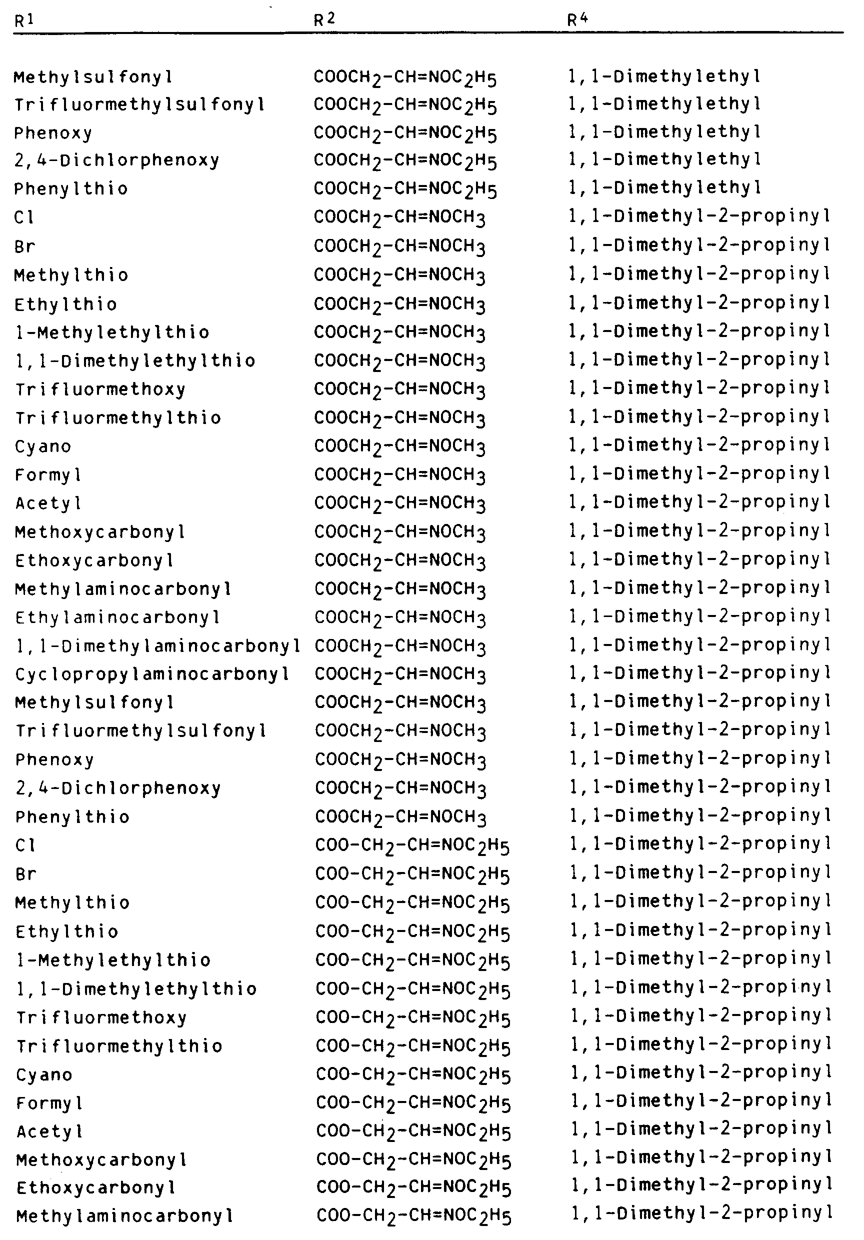

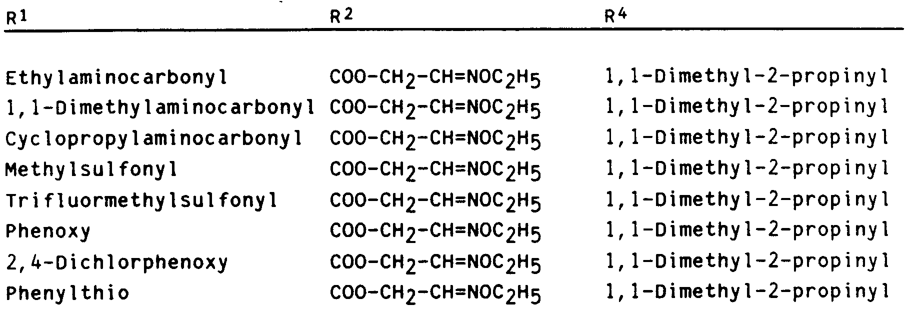

Weiterhin sind von besonderem Interesse die halogensubstituierten Verbindungen I, z.B. 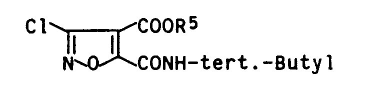
in der R⁵ für Wasserstoff oder folgende Reste steht:
Methyl, Ethyl, Propyl, 1-Methylethyl, 1,1-Dimethylethyl, 2,2,2-Trifluorethyl, 2-Chlorethyl, 2,2,2-Trichlorethyl, 2,3-Dihydroxypropyl, 2-Methoxyethyl, 2,2-Dimethoxyethyl, 2,2,2-Trimethoxyethyl, 2-Methoxy-ethoxymethyl, 2-Methylthioethyl, 2-Methylaminoethyl, 2-Cyclopropylaminoethyl, 2-Dicyclopropylaminoethyl, 2-Trimethylsilylethyl, 2-Methylsulfinylethyl, 2-Methylsulfonylethyl, Carboxymethyl, Methoxycarbonylmethyl, Dimethylaminocarbonylmethyl, Diethoxyphosphonylmethyl, CH₂-CH₂-O-N=C(CH₃)₂, 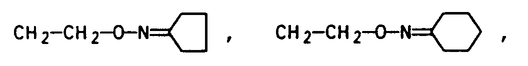
N-Phthalimidomethyl, N-Succinimidomethyl, Benzyloxymethyl, (4-Br-benzoyl)-methyl, (4-Methoxybenzoyl)-methyl, 2-Tetrahydrofuranyl-methyl, 2-Tetrahydrothienylmethyl, 4-Tetrahydropyranyl-methyl, 2-Furanyl-methyl, 2-Thienylmethyl, Benzyl, 2,4-Dichlorbenzyl, 2-Phenylethyl, CH₂-CH=N-OCH₃, CH₂-CH=N-OC₂H₅, CH₂-CH=N-OC₃H₇, CH₂-C(CH₃)=NOCH₃, CH₂-C(CH₃)=NOC₂H₅, CH₂-CH=N-OCH₂CH=CH₂, CH₂-CH=N-OCH₂-C≡CH, 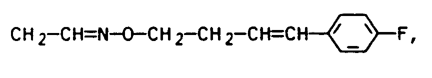
CH₂-CH=NO-C₆H₅, CH₂-CH=N-N-CH₃, CH₂-CH=N-N-C₆H₅, 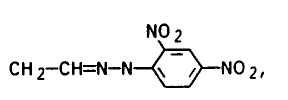
Cyclopentyl, Cyclohexyl, 2-Propenyl, E-CH₂-CH=CH-C₆H₅, 2-Propinyl, 3-Jodpropargyl, 4-Methylphenyl, 4-Methoxyphenyl, 4-Fluorphenyl, 4-Chlorphenyl, 4-Bromphenyl, 4-Cyanophenyl, 4-Nitrophenyl, 2-Chlorphenyl, 2,4-Dimethoxyphenyl, 2,6-Dibrom-4-cyanophenyl, 2-Tetrahydrofuranyl, 2-Tetrahydropyranyl, 1-Pyrazolyl, 1-(1,2,3)-Triazolyl, 1-Benzotriazolyl, Phthalimido, Succinimido, Maleinimido, Na+, K+, NH₄⁺, ⁺NH₃-(1-methylethyl), ⁺NH₂-(1-methylethyl)₂, 2-Propanimino, 2-Butanimino, -N=C(C₂H₅)₂, 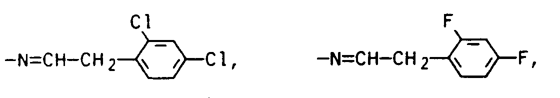
-N=C(Cyclopropyl)₂, Cyclopentanimino, Cyclohexanimino.

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substanten als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. Man vermischt 90 Gewichtsteile der Verbindung 1.001 mit 10 Gewichtsteilen N-Methyl-α-pyrrolidon und erhält eine Lösung, die zur Anwendung in Form kleinster Tropfen geeignet ist.
II. 20 Gewichtsteile der Verbindung 1.009 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung 1.015 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs 1.017 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs 1.005 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs 1.015 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs 1.009 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs 1.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf-oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 2 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

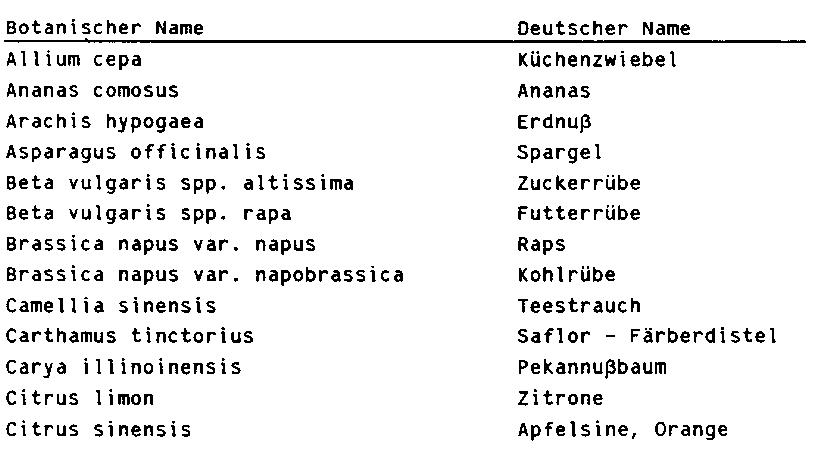

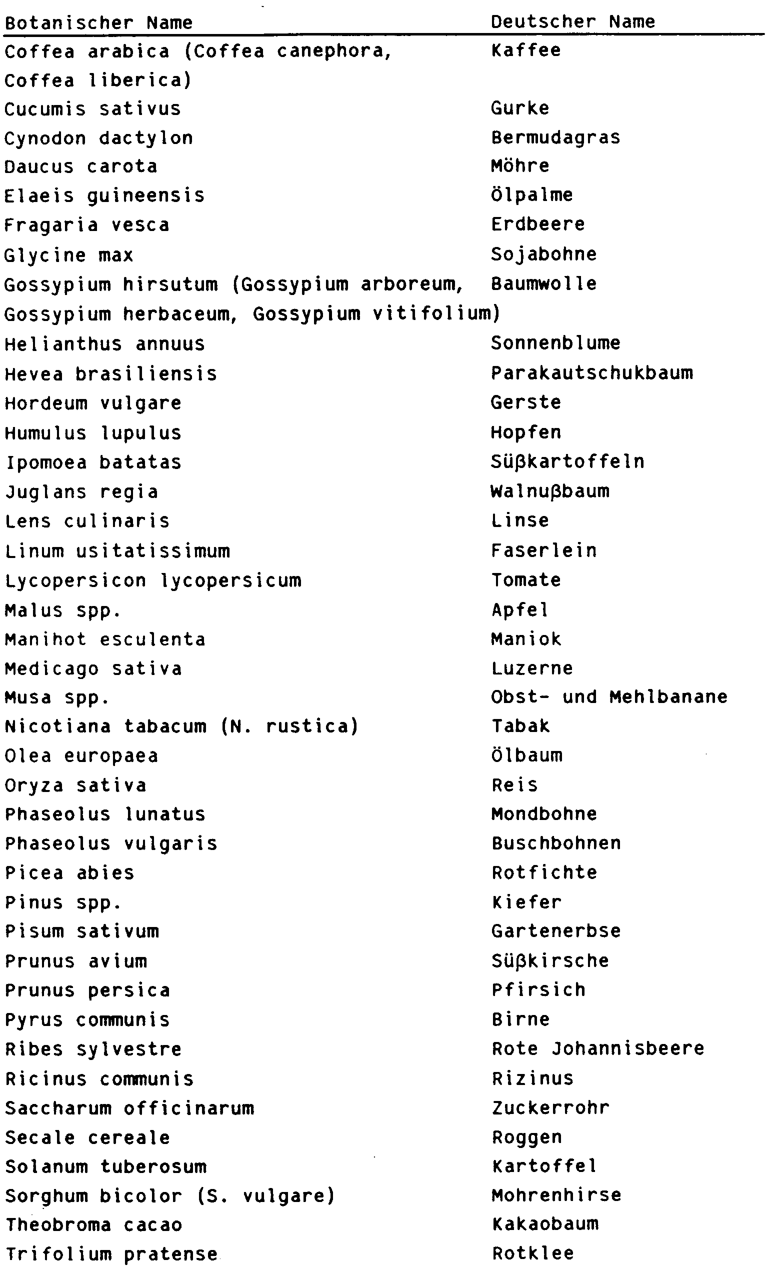

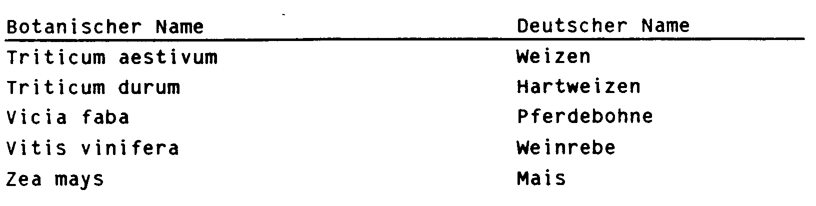

Zur Verbreiterung des Wirkungsspektrums und zur Erzielung synergistischer Effekte können die Verbinungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

### Synthesebeispiele

### 3-tert.-Butylaminocarbonyl-isoxazol-4,5-dicarbonsäuredimethylester

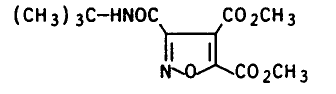

Zu 24,0 g (0,15 mol) tert.-Butylcarbamoylformhydroxamylchlorid und 21,3 g (0,15 mol) Acetylendicarbonsäuredimethylester in 300 ml Tetrahydrofuran tropfte man bei 0°C 18,2 g (0,18 mol) Triethylamin. Man rührte 5 Stunden bei Raumtemperatur und erhitzte 2 Stunden unter Rückfluß. Anschließend zog man das Solvens im Vakuum ab, nahm den Rückstand in 300 ml Dichlormethan auf und extrahierte zweimal mit je 100 ml verd. HCl und einmal mit 100 ml Wasser. Man trocknete die organische Phase über Magnesiumsulfat, zog das Dichlormethan am Rotationsverdampfer ab und reinigte das Rohprodukt durch Säulenchromatographie (SiO₂; Cyclohexan/Ethylacetat 5:1), wobei man den 3-tert.-Butylaminocarbonyl-isoxazol-4,5-dicarbonsäuredimethylester in reiner Form erhielt.

### 3-tert.-Butylaminocarbonyl-5-cyclopropylaminocarbonyl-isoxazol-4-carbonsäuremethylester

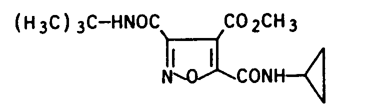

Zu einer Lösung von 2,5 g (8,8 mmol) 3-tert.-Butylaminocarbonyl-isoxazol-4,5-dicarbonsäuredimethylester in 50 ml trockenem Methanol tropfte man bei 20°C unter Kühlung 0,75 g (13,1 mmol) Cyclopropylamin und rührte 20 Stunden bei Raumtemperatur. Man zog die Solventien im Vakuum ab, nahm den Rückstand in Methyl-tert.-butylether auf und extrahierte mit verdünnter HCl und einmal mit Wasser. Anschließend trocknete man die organische Phase über Magnesiumsulfat und engte die Lösung ein. Der so erhaltene 3-tert.-Butylaminocarbonyl-5-cyclopropylaminocarbonyl-isoxazol-4-carbonsäuremethylester wurde mittels Säulenchromatographie gereinigt (SiO₂; Cyclohexan/Ethylacetat 4:1).

### 3-tert.-Butylaminocarbonyl-5-cyclopropylaminocarbonyl-isoxazol-4-carbonsäure

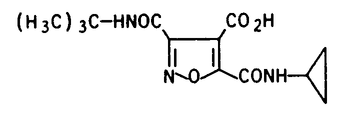

Zu einer Lösung von 1,0 g (3,2 mmol) 3-tert.-Butylaminocarbonyl-5-cyclopropylaminocarbonyl-isoxazol-4-carbonsäuremethylester in 50 ml Methanol wurden bei 5 bis 10°C 0,26 g (6,5 mmol) Natriumhydroxid in 20 ml Wasser gegeben. Nach 12-stündigem Rühren bei Raumtemperatur wurde das Solvens entfernt, der Rückstand in 50 ml Wasser aufgenommen, die Mischung auf pH = 8 bis 9 eingestellt und zweimal mit Diethylether extrahiert. Danach säuerte man die wäßrige Phase auf pH = 2 an, extrahierte mit Ethylacetat und trocknete die vereinigten organischen Phasen über Magnesiumsulfat und entfernte das Lösungsmittel.

In entsprechender Weise wurden die in Tabelle 1 aufgeführten Verbindungen hergestellt.

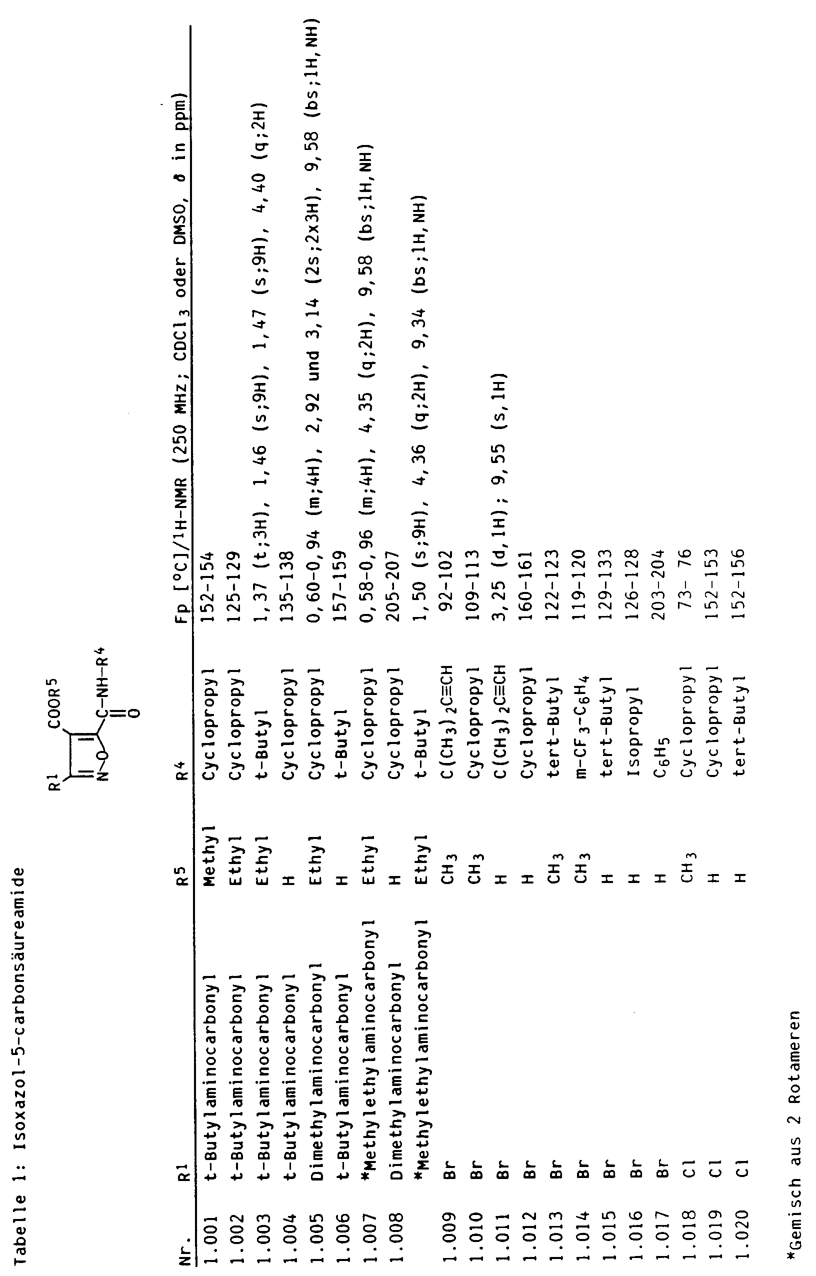

### Anwendungsbeispiele

Die herbizide Wirkung der Verbindungen I ließ sich durch Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 3 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Centaurea cyanus | Kornblume |
| Ipomoea spp. | Prunkwindearten |
| Amaranthus retroflexus | Zurückgekrümmter Fuchsschwanz |

Mit der Verbindung Nr. 1.015 lassen sich bei einer Aufwandmenge von 3 kg/ha im Nachauflaufverfahren breitblättrige Schadpflanzen sehr gut bekämpfen.

## Patentansprüche

1. Isoxazol- und Isothiazol-5-carbonsäureamide der Formel I 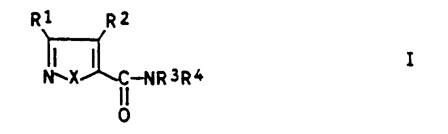 in der die Substituenten folgende Bedeutung haben:
x
Sauerstoff oder Schwefel;
R¹
Halogen,
C₁-C₄-Alkylthio, C₁-C₄-Halogenalkoxy, C₁-C₄-Halogenalkylthio, Cyano, Formyl, C₂-C₄-Alkanoyl, C₁-C₄-Alkoxycarbonyl, C₁-C₄-Alkylaminocarbonyl, Di-(C₁-C₃)-alkylaminocarbonyl, Cyclopropylaminocarbonyl, Methylsulfonyl, Trifluormethylsulfonyl;
Phenoxy oder Phenylthio, welches bis zu dreimal durch C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro substituiert sein kann;
R²
eine Formylgruppe, eine Carbonsäurehalogenidgruppe, eine 4,5-Dihydrooxazol-2-ylgruppe, ein Rest COYR⁵ wobei die Variablen die folgende Bedeutung haben:
Y
Sauerstoff oder Schwefel;
R⁵
Wasserstoff;
eine C₁-C₆-Alkylgruppe₁ welche ein bis fünf Halogenatome und/oder bis zu drei Hydroxy- und/oder C₁-C₄-Alkoxygruppen und/oder einen der folgenden Reste tragen kann:
- Cyano,
- C₁-C₄-Alkoxy-C₂-C₄-alkoxy,
- C₁-C₃-Alkylthio,
- C₁-C₃-Alkylamino, Di-(C₁-C₃)-alkylamino, C₃-C₆-Cycloalkylamino oder Di-(C₃-C₆)-cycloalkylamino,
- Trimethylsilyl,
- C₁-C₃-Alkylsulfinyl oder C₁-C₃-Alkylsulfonyl,
- Carboxyl, C₁-C₃-Alkoxycarbonyl, C₁-C₃-Alkoxycarbonyl-C₁-C₃-alkoxy oder C₁-C₃-Alkoxycarbonyl-C₁-C₃-alkoxy-C₁-C₃-alkoxycarbonyl,
- Di-(C₁-C₃)-alkylaminocarbonyl,
- Di-(C₁-C₃)-alkoxyphosphonyl,
- C₁-C₆-Alkaniminoxy oder C₅-C₆-Cycloalkaniminoxy,
- N-Phthalimido, N-Succinimido, Benzyloxy, Benzoyl, wobei diese cyclischen Reste zusätzlich eine bis drei der folgenden Gruppen tragen können: Halogen C₁-C₃-Alkyl oder C₁-C₃-Alkoxy,
- einen 5- oder 6-gliedrigen gesättigten heterocyclischen Rest oder einen 5- oder 6-gliedrigen heteroaromatischen Rest mit jeweils bis zu 3 Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, C₁-C₃-Alkyl, C₁-C₃-Alkoxy oder C₁-C₃-Alkoxycarbonyl;
- Phenyl, das noch bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy oder C₁-C₂-alkoxycarbonyl;
- einen Rest -CR¹⁰=N-R¹¹, wobei R¹⁰ und R¹¹ die folgende Bedeutung haben:
R¹⁰
Wasserstoff oder C₁-C₆-Alkyl und
R¹¹
C₁-C₆-Alkoxy, C₃-C₆-Alkenyloxy oder C₃-C₆-Alkinyloxy, die jeweils 1 bis 3 Halogenatome und/oder einen Phenylrest mit gewünschtenfalls bis zu drei der folgenden Reste tragen können: Halogen, Nitro, Cyano, C₁-C₃-Alkyl, C₁-C₃-Alkoxy; Phenoxy, das noch bis zu drei der folgenden Substituenten tragen kann: Halogen, Nitro, Cyano, C₁-C₃-Alkyl oder C₁-C₃-Alkoxy; C₁-C₆-Alkylamino, Di-(C₁-C₆)-alkylamino oder Phenylamino, wobei der Phenylrest zusätzlich bis zu drei der folgenden Reste tragen kann: Halogen, Nitro, Cyano, C₁-C₃-Alkyl oder C₁-C₃-Alkoxy;
R⁵ ferner:
C₃-C₈-Cycloalkyl;
C₃-C₆-Alkenyl, C₅-C₆-Cycloalkenyl, C₃-C₆-Alkinyl, wobei diese Reste eine der folgenden Gruppen tragen können: Hydroxy, Halogen, C₁-C₄-Alkoxy oder Phenyl, wobei der Aromat seinerseits eine bis drei der folgenden Gruppen tragen kann: Halogen, Nitro, Cyano, C₁-C₄-Alkyl, partiell oder vollständig halogeniertes C₁-C₄-Alkyl oder C₁-C₄-Alkoxy;
Phenyl, das eine bis drei der folgenden Gruppen tragen kann: Halogen, Nitro, Cyano, C₁-C₄-Alkyl partiell oder vollständig halogeniertes C₁-C₄-Alkyl, C₁-C₄-Alkoxy partiell oder vollständig halogeniertes C₁-C₄-Alkoxy oder C₁-C₄-Alkoxycarbonyl;
einen fünf- oder sechsgliedrigen heterocyclischen Rest mit bis zu drei Heteroatomen, ausgewählt aus der Gruppe Sauerstoff, Schwefel und Stickstoff, wobei zwei Sauerstoff- und/oder Schwefelatome nicht direkt benachbart sein können und wobei die Heterocyclen noch einen oder zwei der folgenden Substituenten tragen können: Halogen, C₁-C₃-Alkyl, C₁-C₃-Alkoxy oder C₁-C₃-Alkoxycarbonyl;
einen Benzotriazolrest;
N-Phthalimido, Tetrahydrophthalimido, Succinimido, Maleinimido;
die 2,2-Dimethyl-1,3-dioxolan-4-ylmethyl- oder 1,3-Dioxolan-2-on-4-ylmethylgruppe;
im Falle Y = O: ein Äquivalent eines Kations aus der Gruppe der Alkali- und Erdalkalimetalle, Mangan, Kupfer, Eisen, Ammonium und mit bis zu 4 C₁-C₃-Alkylgruppen substituiertes Ammonium; oder
ein Rest -N=CR⁸R⁹, wobei
R⁸, R⁹
Wasserstoff; C₁-C₄-Alkyl, das unsubstituiert oder partiell oder vollständig halogeniert sein und einen C₁-C₃-Alkoxy- oder Phenylrest tragen kann, wobei der Phenylrest seinerseits noch ein bis dreimal durch Halogen, Nitro, Cyano, C₁-C₃-Alkyl, partiell oder Vollständig halogeniertes C₁-C₃-Alkyl, C₁-C₃-Alkoxy oder partiell oder vollständig halogeniertes C₁-C₃-Alkoxy substituiert sein kann; C₃-C₆-Cycloalkyl; C₁-C₄-Alkoxy; Furanyl oder Phenyl, das zusätzlich bis zu drei der folgenden Substituenten tragen kann: Halogen, Nitro, Cyano, C₁-C₃-Alkyl, partiell oder vollständig halogeniertes C₁-C₃-Alkyl, C₁-C₃-Alkoxy oder partiell oder vollständig halogeniertes C₁-C₃-Alkoxy; oder R⁸ und R⁹ gemeinsam eine Methylenkette mit 4 bis 7 Gliedern bedeuten;
einen Rest -W-Z, wobei W eine C₂-C₄-Alkylenkette, eine Ethoxyethylenkette, eine But-2-enylen- oder eine But-2-inylenkette bedeutet und Z einen in ω-Stellung an W gebundenen Molekülteil, der den gleichen Molekülteil darstellt, der in α-Stellung von W mit W verknüpft ist, bedeutet;
R³
Wasserstoff;
C₁-C₆-Alkyl, das einen bis drei der folgenden Substituenten tragen kann: Hydroxy, Halogen, C₁-C₄-Alkoxy, C₁-C₄-Alkylthio oder Di-(C₁-C₄)-alkylamino;
C₃-C₈-Cycloalkyl, das ein- bis dreimal durch Halogen, C₁-C₄-Alkyl und partiell oder vollständig halogeniertes C₁-C₄-Alkyl substituiert sein kann;
R⁴
Wasserstoff; Hydroxyl, eine C₁-C₄-Alkoxygruppe;
eine C₁-C₆-Alkylgruppe, die einen bis drei der folgenden Reste tragen kann: Halogen, Cyano, C₁-C₄-Alkoxy, partiell oder vollständig halogeniertes C₁-C₄-Alkoxy, C₁-C₄-Alkylthio, partiell oder vollständig halogeniertes C₁-C₄-Alkylthio, Di-C₁-C₄-alkylamino, C₃-C₈-Cycloalkyl oder Phenyl, wobei der Phenylring seinerseits einen bis drei der folgenden Reste tragen kann: Halogen, Cyano, Nitro, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
eine C₃-C₈-Cycloalkylgruppe, die einen bis drei der folgenden Reste tragen kann: Halogen, Nitro, Cyano, C₁-C₆-Alkyl, partiell oder vollständig halogeniertes C₁-C₆-Alkyl, C₁-C₄-Alkoxy oder partiell oder vollständig halogeniertes C₁-C₄-Alkoxy;
eine C₃-C₆-Alkenylgruppe, deren Doppelbindung epoxidiert sein kann, oder eine C₃-C₆-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
eine Di-(C₁-C₄)-alkylaminogruppe;
ein 5- bis 6-gliedriger heterocyclischer gesättigter oder aromatischer Rest mit einem oder zwei Heteroatomen, ausgewählt aus der Gruppe Sauerstoff, Schwefel und Stickstoff, der ein- bis dreimal durch C₁-C₄-Alkyl oder Halogen substituiert sein kann;
eine Phenylgruppe, die eine bis vier der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Nitro, Cyano, Formyl, C₂-C₄-Alkanoyl, C₂-C₄-Halogenalkanoyl oder C₁-C₄-Alkoxycarbonyl;
eine Naphthylgruppe, die ein- bis dreimal durch C₁-C₄-Alkyl oder Halogen substituiert sein kann;
oder
R³, R⁴
gemeinsam eine Methylenkette mit 4 bis 7 Gliedern, welche durch Sauerstoff, Schwefel oder N-Methyl unterbrochen sein kann, oder den Rest -(CH₂)₃-CO-
sowie die umweltverträglichen Salze der Verbindungen I.

2. Carbonsäureamide der Formel I nach Anspruch 1, wobei X Sauerstoff und R³ Wasserstoff bedeuten.

3. Verfahren zur Herstellung von Verbindungen der Formel I gemäß Anspruch 1, dadurch gekennzeichnet, daß man Isoxazol- bzw. Isothiazol-4,5-dicarbonsäuredialkylester der Formel II 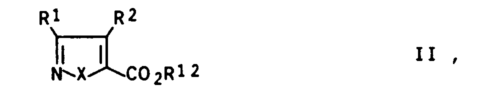 in der R¹² für einen niedermolekularen Alkylrest steht und R¹ und X die in Anspruch 1 genannte Bedeutung haben, zu den Monocarbonsäuren III 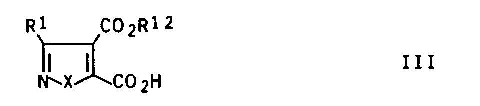 hydrolysiert, die Carboxylgruppe in III in an sich bekannter Weise aktiviert und mit einem Amin HNR³R⁴ umsetzt zum Amid I 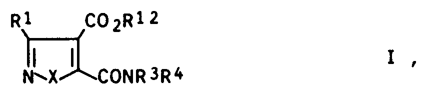 ggf. die Estergruppe in 4-Position durch basische Hydrolyse in die Carboxylgruppe überführt und diese ggf. weiter derivatisiert zum Säurehalogenid oder Ester.

4. Mittel, enthaltend inerte Trägerstoffe und eine herbizid wirksame Menge mindestens eines Carbonsäureamides der Formel I gemäß Anspruch 1.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Carbonsäureamids der Formel I gemäß Anspruch 1 behandelt.

## Claims

1. An isoxazole- or isothiazole-5-carboxamide of the formula I 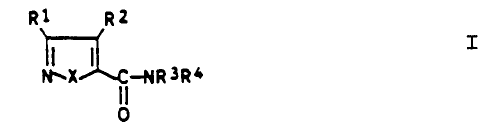 where
X
is oxygen or sulfur;
R¹
is halogen,
C₁-C₄-alkylthio, C₁-C₄-haloalkoxy, C₁-C₄-haloalkylthio, cyano, formyl, C₂-C₄-alkanoyl, C₁-C₄-alkoxycarbonyl, C₁-C₄-alkylaminocarbonyl, di-(C₁-C₃)-alkylaminocarbonyl, cyclopropylaminocarbonyl, methylsulfonyl or trifluoromethylsulfonyl;
phenoxy or phenylthio, which can be substituted up to three times by C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
R²
is formyl, haloformyl, 4,5-dihydro-2-oxazolyl, COYR⁵ where
Y
is oxygen or sulfur;
R⁵
is hydrogen;
C₁-C₆-alkyl which can carry one to five halogens and/or up to three hydroxyl and/or C₁-C₄-alkoxy groups and/or one of the following:
- cyano,
- C₁-C₄-alkoxy-C₂-C₄-alkoxy,
- C₁-C₃-alkylthio,
- C₁-C₃-alkylamino, di-(C₁-C₃)-alkylamino, C₃-C₆-cycloalkylamino or di-(C₃-C₆)-cycloalkylamino,
- trimethylsilyl,
- C₁-C₃-alkylsulfinyl or C₁-C₃-alkylsulfonyl,
- carboxyl, C₁-C₃-alkoxycarbonyl, C₁-C₃-alkoxycarbonyl-C₁-C₃-alkoxy or C₁-C₃-alkoxycarbonyl-C₁-C₃-alkoxy-C₁-C₃-alkoxycarbonyl,
- di-(C₁-C₃)-alkylaminocarbonyl,
- di-(C₁-C₃)-alkoxyphosphonyl,
- C₁-C₆-alkaniminoxy or C₅-C₆-cycloalkaniminoxy,
- N-phthalimido, N-succinimido, benzyloxy, benzoyl, it being possible for these cyclic radicals additionally to carry one to three of the following: halogen, C₁-C₃-alkyl or C₁-C₃-alkoxy,
- a 5- or 6-membered saturated heterocyclic radical or a 5- or 6-membered heteroaromatic radical with, in each case, up to 3 hetero atoms selected from the group comprising oxygen, sulfur and nitrogen, where two oxygen and/or sulfur atoms must not be directly adjacent and where the heterocycles can also carry one or two of the following substituents: halogen, C₁-C₃-alkyl, C₁-C₃-alkoxy or C₁-C₃-alkoxycarbonyl;
- phenyl which can also carry up to three of the following substituents: halogen, nitro, cyano, C₁-C₃-alkyl, partially or completely halogenated C₁-C₃-alkyl, C₁-C₃-alkoxy or partially or completely halogenated C₁-C₃-alkoxy or C₁-C₂-alkoxycarbonyl;
- -CR¹⁰=N-R¹¹ where
R¹⁰
is hydrogen or C₁-C₆-alkyl and
R¹¹
is C₁-C₆-alkoxy, C₃-C₆-alkenyloxy or C₃-C₆-alkynyloxy, each of which can carry 1 to 3 halogens and/or one phenyl with, if required, up to three of the following: halogen, nitro, cyano, C₁-C₃-alkyl, C₁-C₃-alkoxy; phenoxy which can also carry up to three of the following substituents: halogen, nitro, cyano, C₁-C₃-alkyl or C₁-C₃-alkoxy; C₁-C₆-alkylamino, di-(C₁-C₆)-alkylamino or phenylamino where the phenyl can additionally carry up to three of the following: halogen, nitro, cyano, C₁-C₃-alkyl or C₁-C₃-alkoxy;
R⁵
is also C₃-C₈-cycloalkyl;
C₃-C₆-alkenyl, C₅-C₆-cycloalkenyl or C₃-C₆-alkynyl, it being possible for these radicals to carry one of the following groups: hydroxyl, halogen, C₁-C₄-alkoxy or phenyl, the latter possibly carrying one to three of the following groups: halogen, nitro, cyano, C₁-C₄-alkyl, partially or completely halogenated C₁-C₄-alkyl or C₁-C₄-alkoxy;
phenyl which can carry one to three of the following groups: halogen, nitro, cyano, C₁-C₄-alkyl, partially or completely halogenated C₁-C₄-alkyl, C₁-C₄-alkoxy, partially or completely halogenated C₁-C₄-alkoxy or C₁-C₄-alkoxycarbonyl;
a five- or six-membered heterocyclic radical with up to three hetero atoms selected from the group comprising oxygen, sulfur and nitrogen, where two oxygen and/or sulfur atoms must not be directly adjacent and where the heterocycles can also carry one or two of the following substituents: halogen, C₁-C₃-alkyl, C₁-C₃-alkoxy or C₁-C₃-alkoxycarbonyl;
benzotriazolyl;
N-phthalimido, tetrahydrophthalimido, succinimido, maleimido;
2,2-dimethyl-1,3-dioxolan-4-ylmethyl or 1,3-dioxolan-2-on-4-ylmethyl;
where Y is O: one equivalent of a cation from the group comprising alkali metals and alkaline earth metals, manganese, copper, iron, ammonium and ammonium substituted by up to 4 C₁-C₃-alkyl groups; or
-N=CR⁸R⁹ where
R⁸ and R⁹
are each hydrogen; C₁-C₄-alkyl which can be unsubstituted or partially or completely halogenated and can carry a C₁-C₃-alkoxy or phenyl radical, it being possible for the latter in turn to be substituted one to three times by halogen, nitro, cyano, C₁-C₃-alkyl, partially or completely halogenated C₁-C₃-alkyl, C₁-C₃-alkoxy or partially or completely halogenated C₁-C₃-alkoxy; C₃-C₆-cycloalkyl; C₁-C₄-alkoxy; furanyl or phenyl, which can additionally carry up to three of the following substituents: halogen, nitro, cyano, C₁-C₃-alkyl, partially or completely halogenated C₁-C₃-alkyl, C₁-C₃-alkoxy or partially or completely halogenated C₁-C₃-alkoxy; or R⁸ and R⁹ together form a methylene chain with 4 to 7 members;
-W-Z where W is a C₂-C₄-alkylene chain, an ethoxyethylene chain, a 2-butenylene or a 2-butynylene chain, and Z is a moiety which is bonded in the ω-position to W and is the same moiety which is linked to W in the α-position of W;
R³
is hydrogen;
C₁-C₆-alkyl which can carry one to three of the following substituents: hydroxyl, halogen, C₁-C₄-alkoxy, C₁-C₄-alkylthio or di-(C₁-C₄)-alkylamino;
C₃-C₈-cycloalkyl which can be substituted one to three times by halogen, C₁-C₄-alkyl and partially or completely halogenated C₁-C₄-alkyl,
R⁴
is hydrogen; hydroxyl, C₁-C₄-alkoxy;
C₁-C₆-alkyl which can carry one to three of the following: halogen, cyano, C₁-C₄-alkoxy, partially or completely halogenated C₁-C₄-alkoxy, C₁-C₄-alkylthio, partially or completely halogenated C₁-C₄-alkylthio, di-C₁-C₄-alkylamino, C₃-C₈-cycloalkyl or phenyl, it being possible for the latter in turn to carry one to three of the following: halogen, cyano, nitro, C₁-C₄-alkyl, partially or completely halogenated C₁-C₄-alkyl, C₁-C₄-alkoxy, partially or completely halogenated C₁-C₄-alkoxy or C₁-C₄-alkylthio;
C₃-C₈-cycloalkyl which can carry one to three of the following: halogen, nitro, cyano, C₁-C₆-alkyl, partially or completely halogenated C₁-C₆-alkyl, C₁-C₄-alkoxy or partially or completely halogenated C₁-C₄-alkoxy;
C₃-C₆-alkenyl whose double bond can be epoxidized, or C₃-C₆-alkynyl, each of which can be substituted one to three times by halogen and/or once by phenyl which in turn can carry one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
di-(C₁-C₄)-alkylamino;
a 5- to 6-membered heterocyclic saturated or aromatic radical with one or two hetero atoms selected from the group comprising oxygen, sulfur and hydrogen, which can be substituted once to three times by C₁-C₄-alkyl or halogen;
phenyl which can carry one to four of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, nitro, cyano, formyl, C₂-C₄-alkanoyl, C₂-C₄-haloalkanoyl or C₁-C₄-alkoxycarbonyl;
naphthyl which can be substituted once to three times by C₁-C₄-alkyl or halogen;
or
R³ and R⁴
together form a methylene chain with 4 to 7 members, which can be interrupted by oxygen, sulfur or N-methyl, or -(CH₂)₃-CO-
and the environmentally compatible salts of the compound I.

2. A carboxamide of the formula I as claimed in claim 1, where X is oxygen and R³ is hydrogen.

3. A process for preparing a compound of the formula I as claimed in claim 1, which comprises hydrolyzing a dialkyl isoxazole- or isothiazole-4,5-dicarboxylate of the formula II 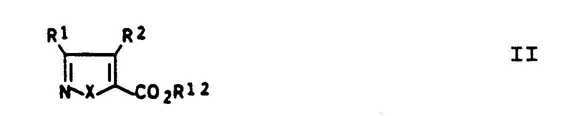 where R¹² is lower alkyl, and R¹ and X have the meanings specified in claim 1, to the monocarboxylic acids III 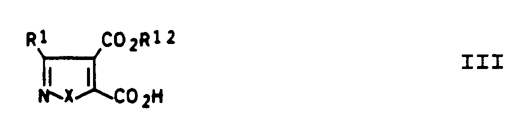 activating the carboxyl in III in a conventional manner and reacting it with an amine HNR³R⁴ to give the amide I 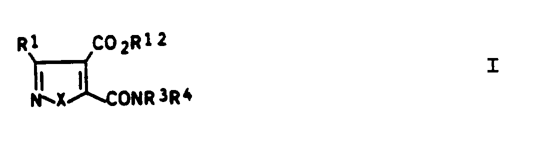 where appropriate converting the ester moiety in position 4 by basic hydrolysis into the carboxyl group and, where appropriate, further derivatizing the latter to give the acid halide or ester.

4. An agent containing inert carriers and a herbicidal amount of at least one carboxamide of the formula I as claimed in claim 1.

5. A method for controlling unwanted plant growth, which comprises treating the unwanted plants and/or their habitat with a herbicidal amount of a carboxamide of the formula I as claimed in claim 1.

## Revendications

1. Amides d'acide isoxazole- et isothiazole-5-carboxylique de formule I 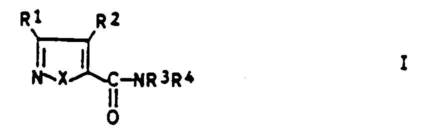 où les substituants représentent les atomes ou groupes suivants:
X
oxygène ou soufre;
R¹
halogène,
alkylthio en C₁-C₄, halogénoalcoxy en C₁-C₄, halogénoalkylthio en C₁-C₄, cyano, formyle, alcanoyle en C₂-C₄, alcoxy(C₁-C₄)carbonyle, alkyl(C₁-C₄)aminocarbonyle, dialkyl(C₁-C₃)aminocarbonyle, cyclopropylaminocarbonyle, méthylsulfonyle, trifluorométhylsulfonyle;
phénoxy ou phénylthio, qui peut être substitué une à trois fois par un groupe alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, halogéno, cyano ou nitro;
R²
un groupe formyle, un groupe halogénure d'acide carboxylique, un groupe 4,5-dihydro-oxazole-2-yle, un reste COYR⁵, où les variables ont la signification suivante:
Y
oxygène ou soufre;
R⁵
hydrogène;
un groupe alkyle en C₁-C₆, qui peut porter un à cinq atomes d'halogène et/ou jusqu'à trois groupes hydroxy et/ou alcoxy en C₁-C₄ et/ou l'un des restes suivants:
- cyano,
- alcoxy(C₁-C₄)-alcoxy(C₂-C₄),
- alkyl(C₁-C₃)thio,
- alkyl(C₁-C₃)amino, di-alkyl(C₁-C₃)amino, cycloalkyl(C₃-C₆)amino ou di-cycloalkyl(C₃-C₆)amino,
- triméthylsilyle,
- alkyl(C₁-C₃)sulfinyle ou alkyl(C₁-C₃)sulfonyle,
- carboxyle, alcoxy(C₁-C₃)carbonyle, alcoxy(C₁-C₃)carbonyl-alcoxy(C₁-C₃) ou alcoxy(C₁-C₃)carbonyl-alcoxy(C₁-C₃)alcoxy(C₁-C₃) carbonyle,
- di-alkyl(C₁-C₃)aminocarbonyle,
- di-alcoxy(C₁-C₃)phosphonyle,
- alcane(C₁-C₆)iminoxy ou cycloalcane(C₅-C₆)iminoxy,
- N-phtalimido, N-succinimido, benzyloxy, benzoyle, tandis que ces restes cycliques peuvent porter en outre un à trois des groupes suivants: halogène, alkyle en C₁-C₃ ou alcoxy en C₁-C₃,
- un reste hétérocyclique saturé à 5 ou 6 chaînons ou un reste hétéroaromatique à 5 ou 6 chaînons ayant à chaque fois jusqu'à 3 hétéroatomes, choisi dans le groupe comprenant l'oxygène, le soufre et l'azote, tandis que deux atomes d'oxygène et/ou de soufre ne peuvent pas être directement voisins et que les hétérocycles peuvent porter encore un ou deux des substituants suivants: halogène, alkyle en C₁-C₃, alcoxy en C₁-C₃ ou alcoxy(C₁-C₃)carbonyle;
- phényle, qui peut encore porter jusqu'à trois des substituants suivants: halogène, nitro, cyano, alkyle en C₁-C₃, alkyle en C₁-C₃ partiellement ou totalement halogéné, alcoxy en C₁-C₃ ou alcoxy en C₁-C₃ partiellement ou totalement halogéné ou alcoxy(C₁-C₂)carbonyle;
- un reste -CR¹⁰=N-R¹¹, où R¹⁰ et R¹¹ ont la signification suivante:
R¹⁰
hydrogène ou alkyle en C₁-C₆ et
R¹¹
alcoxy en C₁-C₆, alcényloxy en C₃-C₆ ou alcynyloxy en C₃-C₆, qui peuvent porter dans chaque cas 1 à 3 atomes d'halogène et/ou un reste phényle ayant en option jusqu'à trois des restes suivants: halogène, nitro, cyano, alkyle en C₁-C₃, alcoxy en C₁-C₃; phénoxy, qui peut encore porter jusqu'à trois des substituants suivants: halogène, nitro, cyano, alkyle en C₁-C₃ ou alcoxy en C₁-C₃; alkyl(C₁-C₆)amino, di-alkyl(C₁-C₆)amino ou phénylamino, tandis que le reste phényle peut en outre porter jusqu'à trois des restes suivants: halogène, nitro, cyano, alkyle en C₁-C₃ ou alcoxy en C₁-C₃;
R⁵ en outre:
cycloalkyle en C₃-C₈;
alcényle en C₃-C₆, cycloalcényle en C₅-C₆, alcynyle en C₃-C₆, tandis que les restes peuvent porter l'un des groupes suivants: hydroxy, halogène, alcoxy en C₁-C₄ ou phényle, le composant aromatique pouvant quant à lui porter un à trois des groupes suivants: halogène, nitro, cyano, alkyle en C₁-C₄, alkyle en C₁-C₄ partiellement ou totalement halogéné ou alcoxy en C₁-C₄;
phényle, qui peut porter un à trois des groupes suivants: halogène, nitro, cyano, alkyle en C₁-C₄, alkyle en C₁-C₄ partiellement ou totalement halogéné, alcoxy en C₁-C₄, alcoxy en C₁-C₄ partiellement ou totalement halogéné, ou alcoxy(C₁-C₄) carbonyle;
un reste hétérocyclique à cinq ou six chaînons ayant jusqu'à trois hétéroatomes, choisis dans le groupe comprenant l'oxygène, le soufre et l'azote, tandis que deux atomes d'oxygène et/ou de soufre ne peuvent pas être directement voisins et les hétérocycles pouvant encore porter un ou deux des substituants suivants: halogène, alkyle en C₁-C₃, alcoxy en C₁-C₃ ou alcoxy(C₁-C₃)carbonyle;
un reste benzotriazole;
N-phtalimido, tétrahydrophtalimido, succinimido, maléinimido;
le groupe 2,2-diméthyl-1,3-dioxolanne-4-ylméthyle ou 1,3-dioxolanne-2-one-4-ylméthyle;
dans le cas où Y = 0: un équivalent d'un cation du groupe comprenant les métaux alcalins et alcalino-terreux, le manganèse, le cuivre, le fer, l'ammonium et l'ammonium substitué par jusqu'à 4 groupes alkyle en C₁-C₃; ou
un reste -N=CR⁸R⁹, où
R⁸, R⁹ désignent les atomes ou groupes suivants:
hydrogène; alkyle en C₁-C₄, qui peut être non substitué ou partiellement ou totalement halogéné et peut porter un reste alcoxy en C₁-C₃ ou phényle, tandis que le reste phényle peut quant à lui encore être substitué une à trois fois par un radical halogène, nitro, cyano, alkyle en C₁-C₃, alkyle en C₁-C₃ partiellement ou totalement halogéné, alcoxy en C₁-C₃ ou alcoxy en C₁-C₃ partiellement ou totalement halogéné; cycloalkyle en C₃-C₆; alcoxy en C₁-C₄; furannyle ou phényle, qui peut en outre porter jusqu'à trois des substituants suivants: halogène, nitro, cyano, alkyle en C₁-C₃, alkyle en C₁-C₃ partiellement ou totalement halogéné, alcoxy en C₁-C₃ ou alcoxy en C₁-C₃ partiellement ou totalement halogéné; ou R⁸ et R⁹ représentent ensemble une chaîne méthylène ayant 4 à 7 chaînons;
un reste -W-Z, où W représente une chaîne alkylène en C₂-C₄, une chaîne éthoxyéthylène, une chaîne but-2-énylène ou une chaîne but-2-ynylène et Z désigne une fraction de molécule liée à W en position ω, représentant la même fraction moléculaire qui est reliée avec W en position α de W;
R³ hydrogène;
alkyle en C₁-C₆, qui peut porter un à trois des substituants suivants: hydroxy, halogène, alcoxy en C₁-C₄, alkylthio en C₁-C₄ ou di-alkyl(C₁-C₄)amino;
cycloalkyle en C₃-C₈, qui peut être substitué une à trois fois par un radical halogène, alkyle en C₁-C₄ et alkyle en C₁-C₄ partiellement ou totalement halogéné;
R⁴
hydrogène; hydroxyle, un groupe alcoxy en C₁-C₄;
un groupe alkyle en C₁-C₆, qui peut porter un à trois des restes suivants: halogène, cyano, alcoxy en C₁-C₄, alcoxy en C₁-C₄ partiellement ou totalement halogéné, alkylthio en C₁-C₄, alkylthio en C₁-C₄ partiellement ou totalement halogéné, di-alkyl(C₁-C₄)amino, cycloalkyle en C₃-C₈ ou phényle, le cycle phényle pouvant quant à lui porter un à trois des restes suivants: halogène, cyano, nitro, alkyle en C₁-C₄, alkyle en C₁-C₄ partiellement ou totalement halogéné, alcoxy en C₁-C₄, alcoxy en C₁-C₄ partiellement ou totalement halogéné ou alkylthio en C₁-C₄;
un groupe cycloalkyle en C₃-C₈, qui peut porter un à trois des restes suivants: halogène, nitro, cyano, alkyle en C₁-C₆, alkyle en C₁-C₆ partiellement ou totalement halogéné, alcoxy en C₁-C₄, alcoxy en C₁-C₄ partiellement ou totalement halogéné;
un groupe alcényle en C₃-C₆, dont la double liaison peut être époxydée, ou un groupe alcynyle en C₃-C₆, qui peuvent être substitués dans chaque cas une a trois fois par un halogène et/ou une fois par un radical phényle, le reste phényle pouvant quant à lui porter un à trois des groupes suivants: alkyle en C₁-C₄, halogénoalkle en C₁-C₄, alcoxy en C₁-C₄, halogénoalcoxy en C₁-C₄, alkylthio en C₁-C₄, halogénoalkylthio en C₁-C₄, halogène, cyano ou nitro;
un groupe di-alkyl(C₁-C₄)amino;
un reste hétérocyclique à 5 à 6 chaînons saturé ou aromatique, ayant un ou deux hétéroatomes, choisis dans le groupe comprenant l'oxygène, le soufre et l'azote, et qui peut être substitué une à trois fois par un radical alkyle en C₁-C₄ ou un halogène;
un groupe phényle, qui peut porter un à quatre des groupes suivants: alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, halogène, nitro, cyano, formyle, alcanoyle en C₂-C₄, halogénoalcanoyle en C₂-C₄ ou alcoxy(C₁-C₄) carbonyle;
un groupe naphtyle, qui peut être substitué une à trois fois par un radical alkyle en C₁-C₄ ou halogène;
ou
R³, R⁴
représentent ensemble une chaîne méthylène ayant 4 à 7 chaînons, qui peut être interrompue par de l'oxygène, du soufre ou un radical N-méthyle, ou le reste -(CH₂)₃-CO-;
ainsi que les sels acceptables pour l'environnement des composés I.

2. Amides d'acide carboxylique de formule I selon la revendication 1, dans lesquels X représente l'oxygène et R³ désigne l'hydrogène.

3. Procédé pour la préparation de composés de formule I selon la revendication 1, caractérise par le fait qu'on hydrolyse un diester alkylique d'acide isoxazole- ou isothiazole-4,5-dicarboxylique de formule II 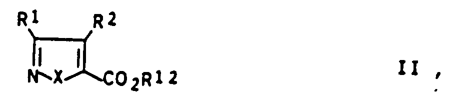 où R¹² désigne un reste alkyle de faible masse moléculaire et R¹ et X ont la signification indiquée dans la revendication 1, pour former les monoacides carboxyliques III 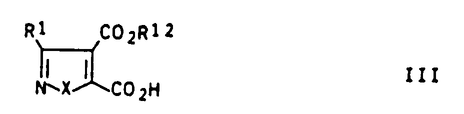 on active de manière connue en soi le groupe carboxyle dans III et on fait réagir avec une amine HNR³R⁴ pour former l'amide I 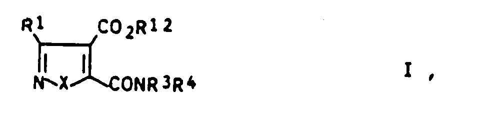 éventuellement on transforme le groupe ester en position 4 par hydrolyse basique en le groupe carboxyle et on désactive ensuite éventuellement celui-ci pour former l'halogénure d'acide ou l'ester.

4. Agent, contenant des supports inertes et une quantité à action herbicide d'au moins un amide d'acide carboxylique de formule I selon la revendication 1.

5. Procédé pour lutter contre la croissance des plantes parasites, caractérisé par le fait qu'on traite les plantes parasites et/ou leur biotope avec une quantité à action herbicide d'un amide d'acide carboxylique de formule I selon la revendication 1.
